(19) Europäisches Patentamt
European Patent Office
Office européen des brevets

(11) EP 1 375 657 A1

(12) **EUROPEAN PATENT APPLICATION**
published in accordance with Art. 158(3) EPC

(43) Date of publication:
**02.01.2004 Bulletin 2004/01**

(21) Application number: **02705141.6**

(22) Date of filing: **13.03.2002**

(51) Int Cl.[7]: **C12N 15/09**, C12Q 1/68, G01N 33/15, G01N 33/50, G01N 37/00

(86) International application number:
**PCT/JP2002/002372**

(87) International publication number:
**WO 2002/077222 (03.10.2002 Gazette 2002/40)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **13.03.2001 JP 2001070940**

(71) Applicant: **Ajinomoto Co., Inc.**
**Tokyo 104-8315 (JP)**

(72) Inventors:
- **YOKOYA, F.,**
**c/o AJINOMOTO Co., Inc.Pharm.Res. Lab**
**Kawasaki-shi, Kanagawa 210-8681 (JP)**
- **OKUTSU, T.,**
**C/O AJINOMOTO Co., Inc.Pharm. Res. Lab**
**Kawasaki-shi, Kanagawa 210-8681 (JP)**
- **MORI, M.,**
**C/O AJINOMOTO Co., Inc. Pharm. Res. Lab.**
**Kawasaki-shi, Kanagawa 210-8681 (JP)**
- **TAKAHARA, Y.,**
**AJINOMOTO Co., Inc. Pharm. Res. Lab.**
**Kawasaki-shi, Kanagawa 210-8681 (JP)**
- **FUKUDA, H.,**
**AJINOMOTO Co., Inc. Pharm. Res. Lab.**
**Kawasaki-shi, Kanagawa 210-8681 (JP)**
- **ABURATANI, Hiroyuki**
**Musashino-shi, Tokyo 180-0003 (JP)**
- **SONAKA, I.,**
**AJINOMOTO Co., Inc. Pharm. Res. Lab.**
**Kawasaki-shi, Kanagawa 210-8681 (JP)**

(74) Representative: **HOFFMANN EITLE**
**Patent- und Rechtsanwälte**
**Arabellastrasse 4**
**81925 München (DE)**

(54) **GENE PANEL PARTICIPATING IN LIVER REGENERATION**

(57) A gene panel comprising a group of genes of which expression levels change in hepatocytes during liver regeneration as compared with those in a normal state is produced by the following steps:

(a) the step of measuring expression levels of various genes in hepatocytes of a model animal in a normal state and expression levels of the genes during liver regeneration;
(b) the step of comparing the expression levels, respectively; and
(c) the step of identifying a group of genes of which expression levels change during liver regeneration.

*Fig. 1*

| | | |
|---|---|---|
| *MboI* | ***CT | 3' |
| | ***GACTAG | 5' |
| *NlaIII* | ***CTCATG | 3' |
| | ***GA | 5' |
| *BfaI* | ***CTC | 3' |
| | ***GAGAT | 5' |
| *HpaII* | ***CTC | 3' |
| | ***GAGGC | 5' |

EP 1 375 657 A1

## Description

### Technical Field

The present invention relates to a gene panel comprising a group of genes of which expression levels change in hepatocytes during liver regeneration as compared with those in a normal state and a method for producing the same as well as use of the gene panel. These aspects of the present invention are useful in the fields of diagnosis, drug and so forth.

### Background Art

After partial hepatectomy in liver cancer treatment or transplantation of partially resected liver in liver disease treatment, rapid regeneration of the remaining liver or transplanted liver is important. However, poor liver regeneration is observed in not a few cases. Further, adults often have fatty livers, and fatty livers often lead to poor regeneration, which causes problems after partial hepatectomy. Furthermore, in order to compensate for damaged liver in hepatitis, it is important to accelerate liver regeneration. Delay in the regeneration worsens hepatitis and can lead to fulminant hepatitis, of which prognosis is poor.

Accordingly, medical care that accelerates liver regeneration is being required, and it can be said that screening for drugs accelerating liver regeneration is important. However, in spite of such requirement, no effective therapeutic agent that accelerates liver regeneration has been found, and no method for efficiently screening for drugs that accelerate liver regeneration has been known.

It is known that, when partial hepatectomy is performed in a rat, liver regeneration is quickly started, and that the hepatocyte population size comes back to the size before the hepatectomy in about 1 week. While it is considered that liver regeneration is accelerated by interactions of two or more kinds of associated genes, the overall picture of liver regeneration process remains unknown for what kinds of genes function at what kinds of timings. Since it is considered that many genes are involved in liver regeneration, it is considered that it is important to examine a large number of important genes involved in liver regeneration to grasp the overall picture of liver regeneration. However, although attempts have been made so far to reproduce liver regeneration in a culture system including a combination of genes and proteins considered to be involved in liver regeneration, no one has succeeded yet. Further, gene screenings utilizing changes in expression observed during liver regeneration as index have been reported (Xu W et al., Biochem. Biophys. Represents. Commun., 278(2): 318-25, 2000; Kar S and Carr BI, Biochem. Biophys. Represents. Commun., 212(1): 21-6, 1995; Mohn KL, et al., Mol. Cell Biol., 11(1): 381-90, 1991; Sobczak J et al., Exp. Cell Res., 169(1): 47-56, 1987; Huber BE et al., Hepatology, 6(2): 209-19, 1986). However, in these screenings, expressions of only a few types of genes have been examined.

### Disclosure of the Invention

The present invention was accomplished in view of the above, and its object is to provide a gene panel comprising genes of which expression levels change in hepatocytes during liver regeneration as compared with those in a normal state and a method for screening for a drug that accelerates liver regeneration by utilizing the gene panel.

The inventors of the present invention considered that liver regeneration could be accelerated by realizing behaviors of genes involved in liver regeneration close to those of their patterns observed when liver regeneration was being accelerated. Then, they examined expression profiles of various genes during liver regeneration by using partially hepatectomized rats to obtain expression information about genes involved in liver regeneration, and thus accomplished the present invention.

That is, the present invention provides the followings.

(1) A gene panel comprising names of genes of which expression levels change in hepatocytes during liver regeneration as compared with those in a normal state and expression profiles of the genes.
(2) The gene panel according to (1), wherein the changes of the gene expression levels are changes in the expression levels in a model animal after partial hepatectomy as compared with the expression levels in a normal state in the model animal.
(3) The gene panel according to (1) or (2), wherein the expression profiles include expression profiles over time during liver regeneration.
(4) The gene panel according to any one of (1) to (3), which includes sequence information of a group of PCR primers for analyzing the expression profiles of the genes.
(5) The gene panel according to any one of (2) to (4), wherein the model animal is a rat.
(6) The gene panel according to any one of (1) to (5), which includes expression profiles of at least 6 types of genes among 166 types of the genes represented by the numbers 1 to 166 in Tables 1. 6 and 7.
(7) The gene panel according to (6), wherein the at least 6 types of genes are selected from 151 types of the genes represented by numbers 1 to 151 in Tables 1 and 6.
(8) The gene panel according to (6), wherein the at least 6 types of genes are selected from 137 types of the genes represented by the numbers 1 to 137 in Tables 1 and 6.
(9) The gene panel according to any one of (6) to (8), wherein the at least 6 types of genes are the genes represented by the numbers 5, 17, 36, 46, 67 and 68 in Table 1.
(10) A method for producing a gene panel comprising expression profiles of genes of which expression levels change in hepatocytes during liver regeneration as compared with those in a normal state, which comprises the steps of:

(a) measuring expression levels of various genes in hepatocytes of a model animal in a normal state and expression levels of the genes during liver regeneration;
(b) comparing the expression levels, respectively; and
(c) identifying a group of genes of which expression levels change during liver regeneration and producing expression profiles from information about gene names and changes in the expression levels.

(11) The method according to (10), wherein, in the step (a), the expression levels of the gene are analyzed over time during liver regeneration.
(12) The method according to (11), wherein a liver regeneration accelerating substance is administered before, during or after liver regeneration.
(13) The method according to (12), wherein the liver regeneration accelerating substance is L-alanine.
(14) The method according to any one of (10) to (13), wherein the gene expression levels are analyzed by one or more kinds of methods selected from the gene chip method, the ATAC-PCR method and the Taqman PCR (SYBR Green) method.
(15) The method according to (14), wherein the gene expression levels are analyzed by the gene chip method and the ATAC-PCR method.
(16) The method according to (14), wherein the gene expression levels are analyzed by the Taqman PCR (SYBR Green) method.
(17) A method for screening for a drug involved in liver regeneration, which comprises administering a drug to a model animal or liver tissue or cells and profiling expressions of genes constituting the gene panel according to (1).
(18) A method for evaluating a condition of liver, which comprises profiling expressions of genes constituting the gene panel according to (1) for liver of a subject.
(19) A group of primers used in the method for producing a gene panel according to (10), the screening method according to (17) or the evaluation method according to (18), which comprises all or a part of the oligonucleotides of SEQ ID NOS: 1 to 127 and 135 to 192.

Hereafter, the present invention will be explained in detail.

<1> Gene panel of the present invention

The gene panel of the present invention is a gene panel comprising names of genes of which expression levels change in hepatocytes during liver regeneration as compared with those in a normal state and gene expression profiles of the genes.
The gene panel of the present invention can be produced by the following steps:

(a) the step of measuring expression levels of genes in hepatocytes of a model animal in a normal state and expression levels of the genes during liver regeneration;
(b) the step of comparing the expression levels, respectively; and
(c) the step of identifying a group of genes of which expression levels change during liver regeneration and producing expression profiles from information about gene names and changes in expression levels.

The liver regeneration refers to a phenomenon that, when a part of cells in the liver are damaged or lost due to resection or the like, the condition is repaired and goes back to the original normal liver condition. For example, this phenomenon is observed in the remaining liver or transplanted liver after partial hepatectomy in liver cancer treatment or transplantation of partially resected liver in liver disease treatment.
The gene expression level is synonymous with expression amount, expression intensity or expression frequency of a gene, and is usually analyzed based on a production amount of a transcription product corresponding to the gene, activity of its translation product or the like.

The gene expression level can be measured by methods usually utilized in analyses of gene expressions. Examples of preferred methods include the gene chip method, the gene microarray method, the gene macroarray method and so forth. In these methods, gene fragments arrayed and immobilized on a certain plate (usually, slide glass) are used. This chip is hybridized with fluorescence-labeled mRNA, and the type and amount of the mRNA are measured.

Further, as methods preferred in the present invention, the ATAC-PCR method and the Taqman PCR (SYBR Green) method can be mentioned. The ATAC-PCR method is a type of the quantitative PCR technique developed by Kato et al (Kato, K. et al., Nucl. Acids Res., 25, 4694-4696, 1997) and is characterized in that it enables quantification of expression of a trace amount sample. Further, while conventional quantitative PCR methods enable quantification of expressions of several tens of types of genes at most, the ATAC-PCR method enables quantification of expressions of 1000 or more types of genes. The Taqman PCR (SYBR Green method) is a common technique for quantitative RT-PCR (Schmittgen TD, Methods, 25, 383-385, 2001) and is characterized in that primers can be readily designed. Moreover, its procedure is simple, and expressions of a large number of genes can be measured in a short time (40 to 150 genes/2 hours).

Besides the above, examples of the gene expression analysis method include the body map method (Gene, 174, 151-158, 1996), serial analysis of gene expression (SAGE) (U.S. Patent Application Nos. 527,154 and 544,861, European Patent Publication No. 0761822), micro-analysis of gene expression (MAGE) (Japanese Patent Laid-open Publication (Kokai) No. 2000-232888) and so forth.

The body map method will be outlined below. A poly-T sequence on a vector and a poly-A tail at the 3' end of mRNA are ligated, and cDNA is synthesized by using the vector poly-T sequence as a primer. Further, the cDNA is digested with a restriction enzyme MboI. Since one MboI site exists per 300 base pairs on average on cDNA, the cDNA on the vector is divided into fragments of 300 base pairs in average. At this time, the cDNA fragment closest to the poly-A tail remains as ligated to the vector. The vector having this cDNA fragment is cyclized and introduced into *Escherichia coli* to construct a cDNA library. About 1000 clones are arbitrarily selected from the library, and a nucleotide sequence of 300 base pairs in average is determined for each clone. Among these sequences, clones comprising the same sequences are collected, and the type and the emerging frequency of each sequence are obtained to create a gene expression profile. Homology search (BLAST search) in the data bank is performed for each cDNA sequence, and a clone having the same sequence as that of a known gene is given the name of the gene. When the sequence is not registered at the data bank, it is assumed that a gene corresponding to the sequence does not exist.

In order to perform the homology search by the BLAST search, information of at least 11 base pairs is necessary. There are about one million types of genes having sequences comprising 10 nucleotides. This number of types is far more than the number of 100,000 types of genes expected to exist in human. That is, if there is information about 11 base pairs, genes having the sequence can be identified, and the gene expression profiles can be analyzed. Therefore, in order to efficiently analyze gene expression profiles by the body map method requiring many sequences, cDNA fragments having about 300 base pairs in the body map can be further divided into short fragments having 11 base pairs or more (referred to as "tag"), and these fragments can be further ligated to one another as many kinds of fragments and inserted into a vector to construct a ligated tag library. Then, if about 1000 clones are arbitrarily selected as in the body map method to determine the DNA sequences of the ligated tags, it can be expected that information about expressions of more genes can be obtained with the same procedure as in the body map method. Each tag represents a gene sequence, and the emerging frequency of the tag represents the expression frequency of the gene. Since the length of a DNA sequence that can be determined in one sequencing is usually about 600 base pairs, DNA sequences of about 50 tags at most can be determined by one sequencing. That is, gene expression profile analysis can be performed with about 50 times higher efficiency at most compared with the body map method.

The SAGE method is a method for analyzing gene expression profiles based on the above concept. The SAGE method is performed as follows. cDNA is prepared by using a poly-T bonded with biotin at the 3' end as a primer and digested with a restriction enzyme such as *MboI* (referred to as "anchoring enzyme") as in the body map, and then a cDNA fragment including the 3' end bonded with biotin is adsorbed on avidin beads. Then, the beads are divided into two portions, and one of two types of linkers (A and B) is bonded to the cDNA fragment (about 13 bp) adsorbed on the beads of one of the two portions, respectively. A site for a Class II restriction enzyme such as *BsmFI* (referred to as "tagging enzyme) is added to each linker beforehand. The cDNA fragments are excised from the beads with the tagging enzyme, the digestion sites are blunt-ended, and the tag ligated to the linker A and the tag ligated to the linker B are ligated to each other. This product is called a ditag. The ditag is amplified by PCR using primers recognizing the linker A and the linker B. The amplified ditags are ligated to one another as many kinds of fragments and incorporated into a vector and sequenced. About 50 tag sequences can be obtained per sequencing. This tag sequence information is collected to obtain the gene expression frequency.

The MAGE method is a modified version of the aforementioned method, and is a method enabling gene expression frequency analysis with high efficiency and precision, in which cDNAs are synthesized from mRNAs by using a vector primer having a poly-T sequence, the cDNA sequences are tagged on a vector, the obtained tags are ligated via a sequence that allows identification of the tag end to form a concatemer, and the nucleotide sequence of the concatemer is analyzed.

In the present invention, the gene expression analysis method is not particularly limited so long as gene expression levels can be analyzed, and any of currently known methods and methods to be developed in future can be employed. Among the methods mentioned above, particularly preferred are the gene chip method, the gene microarray method, the ATAC-PCR method and the Taqman PCR (SYBR Green) method.

In the present invention, gene expression levels may be analyzed based on results obtained by a single kind of method or results obtained by two or more kinds of methods. Although the analysis can be performed by a single kind of method to a certain extent, more precise analysis can be attained by using two or more kinds of methods in combination. Specifically, a correlation coefficient is calculated for results obtained by two or more kinds of methods, for example, the gene chip method and the ATAC-PCR methods. When the correlation coefficient is a certain value or higher, it is assumed that the expression level of the corresponding gene has changed.

Various gene chips, gene microarrays and gene macroarrays of human or animal such as mouse are commercially available, and these may be used in the present invention.

In the present invention, changes in gene expression levels can be analyzed by measuring expression levels of various genes in hepatocytes in a normal state and expression levels of these genes during liver regeneration and comparing their expression levels, respectively. The gene expression levels during liver regeneration are analyzed by, for example, using a model animal such as a partially hepatectomized rat and measuring the gene expression levels in hepatocytes after the hepatectomy. On the other hand, the gene expression levels in a normal state are typically measured immediately before the hepatectomy. The gene expression levels are preferably analyzed over time during liver regeneration.

As described above, a group of genes of which expression levels change during liver regeneration is identified. Expression profiles of these genes are produced by allocating information about the change of analyzed expression level to the identified gene, respectively.

Further, in the present invention, as genes of which expression levels change during liver regeneration as compared with those in a normal state, that is, candidates of genes considered to be involved in liver regeneration, genes estimated to be involved in the metabolism of L-alanine can be mentioned.

L-alanine is known to accelerate liver regeneration (Maezono K et al., Hepatology 24(5), 1996; Japanese Patent Laid-open Publication No. 5-229940). Further, elevation of the L-alanine concentration in human plasma after hepatectomy (Nijveldt RJ et al., Liver 21(1): 56-63, 2001), accumulation of L-alanine in rat liver tissue after hepatectomy (Brand HS et al., J. Hepatology, 23, 333-340, 1995) and so forth have been reported. As data supporting these findings, elevation of mRNA level of the amino acid transporter system A (ATA2), which transports L-alanine, was observed in 70% partially hepatectomized rat as a liver regeneration model, as described later (the examples of the present specification). Further, since increase of the L-alanine transporting activity in a similar liver regeneration model has also been observed (Joan-Vicenc et al., FEBS LETTERS, 329(1,2): 189-193, 1993; Freeman TL et al., BBRC, 278, 729-732, 2000), possibility of increase in uptake of L-alanine into hepatocytes during liver regeneration is suggested.

Further, the uptake of [$^3$H] thymidine into DNA is decreased when the Ala transport activity of ATA2 is inhibited by a treatment with MeAIB (nonmetabolizable System A-specific substrate, alpha-(methylamino)isobutyric acid) (Freeman TL et al., Hepatology, 30(2): 437-444, 1999). Therefore, it is suggested that acceleration of liver regeneration is suppressed when transport of L-alanine into hepatocytes is inhibited. This also strongly suggests that ATA2 (Ala) is involved in acceleration of liver regeneration.

Furthermore, in the field of surgical operations, it has been reported from old days that markedly increased energy metabolism is observed in liver under an invasive condition such as hepatectomy, and amino acids represented by L-alanine are supplied primarily as a result of decomposition of myoproteins and used as substrates for gluconeogenesis (Felig P et al., Metabolism, 22, 179-207, 1973) and protein synthesis. As described above, L-alanine is generally considered to supply energy through gluconeogenesis during liver regeneration. In fact, oxidation of L-alanine was suppressed in a liver regeneration model rat at 9 or 18 hours after hepatectomy, and increase in plasma glucose level, accumulation of liver glycogen and enhanced activities of fructose-1,6-diphosphatase and PEPCK have also been reported (Klain GJ et al., J. Nutr. Biochem., 1 (Nov), 578-584, 1990).

Based on the above findings, it was revealed that L-alanine accelerated liver regeneration, and it was hypothesized that its action mechanism is based on the energy supply increased by L-alanine during liver regeneration. Therefore, as shown in the examples described later, expressions of 59 types of rat genes involved in L-alanine metabolism were analyzed during liver regeneration. As a result, changes in expression were observed for 17 types of the genes. Accordingly, when the gene panel of the present invention is produced, genes involved in the L-alanine metabolism may be selected prior to the step (a) mentioned above.

Even for a gene of which expression change is not observed during liver regeneration, expression change may be observed if L-alanine is administered before, during or after liver regeneration as shown in Example 3. Such a gene may also be included in the gene panel. Further, a substance having an action for accelerating liver regeneration (liver regeneration accelerating substance) other than L-alanine may also be administered before, during or after liver regeneration. Examples of the liver regeneration accelerating substance include a mixture of liver extract and flavin adenine dinucleotide (15 μl of liver extract, 10 mg of FAD per 1 ml), liver hydrolysate, saiko-keishito, shosaikoto, diisopropylamine dichloroacetate, placenta hydrolysate, valine (WO 0113912), vascular endothelial growth factor/vascular permeability factor (VEGF/VPF) (WO 0132213), macrolide compounds (JP 3240728), TCF II (JP 10194986), TGF-β release/activation/synthesis inhibitor (JP8333249), glucose-1-phosphate, glucose-6-phosphate (JP2202821), desialated orosomucoid (JP 6122025) and so forth.

The gene panel of the present invention includes at least names of various genes identified as described above and expression profiles of the respective genes. As for the name of genes, the nomenclature for the genes is not particularly limited so long as the genes can be distinguished from one another. Typically, names of products encoded by the genes, accession numbers or gene names used in the database of GenBank etc., probe set names or gene names on a gene chip and so forth are used.

In the present invention, the term "expression profile" means information about change in expression level of each gene included in the gene panel. In a preferred embodiment, the expression level is measured and recorded over time. In the expression profile, expression level of a gene may be represented by an absolute value or relative value.

In a preferred embodiment of the gene panel of the present invention, genes are classified according to the expression levels at certain times after hepatectomy. For example, genes are classified into a group in which the expression levels markedly increase 1 hour and 6 hours after hepatectomy (early stage of liver regeneration) (Groups 1 and 2), a group in which the expression levels conversely decrease at this stage (Groups 6 and 7), a group in which the expression levels markedly increase 24 hours and 48 hours after hepatectomy (mid stage of liver regeneration) (Groups 3 and 4), a group in which the expression levels conversely decrease at this stage (Groups 8 and 9), a group in which the expression levels markedly increase 7 days after hepatectomy (later stage of liver regeneration) (Group 5) and a group in which the expression levels conversely decrease at this stage (Group 10). The expression "markedly" used herein means that the increase or decrease of the expression level is 3 times more compared with the level before hepatectomy in the gene chip method, or that, in the case of the Taqman-PCR (SYBR Green) method, if a difference is observed in a significance test, it is considered that there is a difference of expression.

In an embodiment of the gene panel of the present invention, these groups can be listed in the order of the length of the time for which the expression levels after the increase or decrease of expression are maintained or for which the increase or decrease continues, starting with the longest time as listed in Table 1.

Further, the gene panel preferably includes information about liver weight at a certain time after hepatectomy. For example, liver is extracted at an early stage of liver regeneration (1 hour and 6 hours after hepatectomy), mid stage of liver regeneration (24 hours and 48 hours after hepatectomy) and later stage of liver regeneration (7 days after hepatectomy), and weight of each liver is measured.

The gene panel of the present invention may include sequence information of PCR primers for analyzing expression level of each gene. Examples of the primers include primers comprising all or a part of the oligonucleotides of SEQ ID NOS: 1 to 127 and 135 to 192. Further, in addition to the sequence information of the primers, nucleotide sequence information of adaptors used in the ATAC-PCR method and/or the Taqman PCR (SYBR Green) method may be included. SEQ ID NOS: 1 to 127 represent primers used in the ATAC-PCR method, and SEQ ID NOS: 135 to 192 represent primers used in the Taqman PCR (SYBR Green) method. As for the nucleotide sequences of the adaptors, adaptors having the nucleotide sequence of SEQ ID NOS: 128 to 133 can be mentioned.

<2> Screening method of the present invention

Based on the gene panel of the present invention, various screening tests are enabled by constructing a system for quantitatively or semi-quantitatively measuring expressions of genes included in the gene panel.

For example, screening for a drug involved in liver regeneration can be performed by administering drugs to a model animal or liver tissue or cells and profiling expressions of genes constituting the gene panel of the present invention. That is, a drug that provides gene expression profiles similar to the expression profiles in the gene panel is considered to accelerate liver regeneration.

Further, a liver regeneration accelerating substance can also be screened by screening for a drug that further increases or decreases the increased or decreased expression levels of genes of the gene panel of the present invention. In this case, screening can be performed by paying attention to expression changes of about 6 types of genes. Examples of the screening based on this conception will be shown in the examples described later.

As the aforementioned 6 types of genes, for example, there can be mentioned the genes of Nos. 5 (amino acid transporter system A (ATA2)), 17 (high mobility group protein 2), 36 (nuclear RNA helicase), 46 (liver nuclear protein p47), 67 (leukemia-associated cytosolic phosphoprotein stathmin) and 68 (deoxy-UTPase (dUTPase)) among the 137 types of genes in the gene panel shown in Table 1 ((1) to (11)).

Examples of the method for profiling gene expressions include the DNA microarray and DNA macroarray methods using a slide glass, nylon membrane or the like on which fragments of genes constituting the gene panel are immobilized, the ATAC-PCR method, a method utilizing the Taqman probe, quantitative PCR utilizing SYBR Green and so forth. The expression profiling may be carried out by using a single kind of method or two or more kinds of methods in combination.

Hereafter, a specific example of screening procedure will be described.

As primary screening, cultured hepatocytes are treated with drugs to be screened or the drugs are administered to rats. After a certain time, RNAs are prepared from the cells or liver. The gene expression levels are measured before and after the drug treatment, and drugs that provide expression profiles similar to those in the gene panel are screened. The gene expression patterns in hepatocytes after the drug treatment are grouped, and drugs that provide grouping similar to the grouping in the gene panel are screened. The grouping is performed by classifying the genes according to expression levels at a certain time in the same manner as in the production of the gene panel.

As for culture conditions of hepatocytes, usual culture may be used, or addition of substances that damage hepatocytes such as galactosamine, heavy metal ions or carbon tetrachloride to a medium and so forth may be used. It is expected that substances that accelerate liver regeneration or proliferation of cultured hepatocytes can be screened by culturing cells under the usual condition. Further, it is expected that substances that accelerate recovery of the liver function by liver regeneration after cell damage can be screened by carrying out the culture in the presence of a substance that damages hepatocytes.

As secondary screening, candidate drugs expected to exhibit action for accelerating liver regeneration based on the results of the primary screening are administered to partially hepatectomized rats, and liver is extracted after certain intervals, for example, at an early stage of liver regeneration (1 hour and 6 hours after hepatectomy), mid stage of liver regeneration (24 hours and 48 hours after hepatectomy) and later stage of liver regeneration (7 days after hepatectomy). The liver weights are measured, and RNAs are extracted from the livers to measure changes in gene expression. The obtained data are compared with data of liver weight changes and gene expression changes in partially hepatectomized rats not administered with the drugs to evaluate effect of the drugs on liver regeneration.

The aforementioned screening can also be performed by using laboratory animals other than rats. In this case, it is preferable to newly produce a gene panel for homologues corresponding to the rat genes and perform the screening.

In addition to the screening of drugs that provide expression changes similar to those in the gene panel, the following drug screening can be performed by utilizing the gene panel. The genes that show the changes in the early stage of liver regeneration (for example, Groups 1, 2, 6 and 7 mentioned above) are considered to be important for initiation of liver regeneration. Therefore, it is expected that drugs effective for the initiation of liver regeneration can be obtained by screening for drugs that provide patterns similar to the patterns of these genes of these groups after the treatment with the drugs.

Further, the genes of which expression changes start in the mid stage of liver regeneration (for example, those of Groups 3, 4, 8 and 9 mentioned above) are considered to act on acceleration of proliferation or differentiation of hepatocytes after the initiation of liver regeneration. Therefore, it is expected that drugs that positively act on the acceleration of proliferation or differentiation of hepatocytes can be obtained by screening for drugs that provide patterns similar to the patterns of these genes after the treatment with the drugs.

The genes of which expression changes start in the later stage of liver regeneration (for example, those of Groups 5 and 10 mentioned above) are considered to be important for termination of liver regeneration and normal hepatocyte functions after the completion of regeneration. Therefore, it is expected that drugs that negatively act on the proliferation of hepatocytes or drugs effective for normal hepatocyte functions after the completion of regeneration can be obtained by screening for drugs that provide patterns similar to the patterns of these genes after the treatment with the drugs. The drugs that negatively act on the proliferation of hepatocytes can be used for prevention of excessive proliferation of hepatocytes, and possibility of use thereof as drugs for liver cancer or the like is expected.

As described above, the gene panel of the present invention is considered to be useful also for screening for drugs effective in each stage of liver regeneration (for example, initiation, cellular proliferation, differentiation, termination of cellular proliferation etc.), and it is considered that more effective liver regeneration agents can also be created by using drugs obtained as a result of the above screenings.

L-alanine is known to have liver regeneration effect. However, since L-alanine is easily metabolized and disappears in a living body, sufficient drug efficacy cannot be obtained. Accordingly, drugs are being required which have the same drug efficacy as that of L-alanine and are imparted with better characteristics such as prolonged effect obtained through improved pharmacokinetics. Further, there are substances such as crude drugs, of which drug efficacy is known but amount is insufficient for use as a drug since it is hard to secure their raw material and they act as a mixture of two or more kinds of components. Therefore, substances that have liver regeneration action and can be sufficiently supplied are being required. The present invention enables screening of substances useful as drugs with high sensitivity and high efficiency.

<3> Method for evaluating condition of liver of the present invention

The gene panel of the present invention can be used for a system for evaluating whether diseased liver shows progression towards regeneration in liver diseases such as hepatitis and cirrhosis. Further, a drug treatment suitable for each patient can be performed by examining the gene expression change pattern of each patient. For example, a series of drugs that specifically control certain genes (gene group) in the gene panel of the present invention in a positive or negative manner are screened. Then, drugs necessary for liver regeneration can be selected by examining expressions of the genes in the gene panel of each patient with liver disease.

Brief Description of the Drawings

Fig. 1 shows a structure of adaptor.

Figs. 2 to 4 show relative expression levels of genes involved in L-alanine metabolism, of which expressions change during liver regeneration after administration of L-alanine (relative expression levels based on the expression level of β-actin, which is taken as 1000).

Best Mode for Carrying out the Invention

The present invention will be explained more specifically with reference to the following examples.

Example 1

<1> Preparation of regenerating liver

Ten week-old F344/DvCrj (Fischer) rats were etherized. The abdomen was incised, and the left lobe and the intermediate lobe of the liver were extruded from the incision. The liver was bound with sutures between the left lobe and the intermediate lobe and between the right lobe and the caudate lobe. The right lobe and the caudate lobe were left in the body, and the left lobe and the intermediate lobe were resected. Then, the incision was closed with a suture. As for the animals which were examined at 0 hour after the excision, suturing was not performed, and the remaining liver was successively extracted. The incision was opened again at 1, 6, 12, 24 and 168 hours after the hepatectomy to extract the remaining liver (regenerating liver). The weight of the remaining livers (right lobe and caudate lobe) was about 30% of the weight of the whole rat liver (right lobe, left lobe, intermediate lobe and caudate lobe).

Three liver regeneration model rats were prepared for each group, and the remaining liver weight at the time of extraction was measured. The weight was 2 ± 0.3 g (1.6 g) 0 hour after the excision, 1.7 ± 0.3 g (1.4 g) 1 hour after the extraction, 2.1 ± 0.5 g (2.1 g) 6 hours after the extraction, 2.5 ± 0.5 g (3.0 g) 24 hours after the extraction, 2.5 ± 0.5 g (2.0 g) 48 hours after the extraction, and 5.6 ± 0.3 g (5.8 g) 7 days after the extraction (the parenthesized numerical values are weights of the regenerating livers used for the measurement using a gene chip described later).

<2> Purification of total RNA

In a volume of 10 ml of ISOGEN (Nippon Gene) was added to 1 g of rat liver, and the liver was homogenized. The obtained homogenate was centrifuged to collect supernatant. This supernatant was added with 200 μl of chloroform per 1 ml of the added ISOGEN and lightly stirred. The mixture was left at room temperature for 2 minutes and centrifuged at 15000 rpm at 4°C for 10 minutes, and the aqueous layer was transferred to a new centrifuge tube. This aqueous layer was added with an equivalent volume of 2-propanol, left at room temperature for 5 minutes and centrifuged at 15000 rpm at 4°C for 15 minutes. The supernatant was discarded, and the precipitated pellet was added with 70% ethanol and centrifuged at 15000 rpm at 4°C for 15 minutes. The pellet was rinsed by removing the 70% ethanol. The rinsed pellet was dried at room temperature for 5 minutes and added with DEPC (diethyl pyrocarbonate)-treated water to dissolve the pellet. Purification of the total RNA fraction obtained as described above was confirmed by 1% agarose gel electrophoresis.

As described above, total RNA was purified from each of the remaining livers (regenerating liver) at 0, 1, 6, 24, 48 and 168 hours after the partial hepatectomy of rats, and changes in expression levels of various genes were examined by using GeneChip (Affymetrix) and ATAC-PCR.

<3> Gene expression analysis by GeneChip

Gene expressions were analyzed by using GeneChip according to the protocol recommended by Affymetrix. The procedure is shown below.

(1) Probe synthesis

(i) Synthesis of double-stranded cDNA

First, double-stranded cDNA was synthesized from the total RNA prepared in <2> by using SUPERSCRIPT Choice System produced by Gibco BRL. In an amount of 15 μg of total RNA and 100 pmol of T7-$(dT)_{24}$ primer were dissolved in DEPC-treated water to a volume of 11 μl. A reaction was allowed at 70°C for 10 minutes, and then the reaction mixture was cooled on ice, added with 4 μl of 5 x 1st strand cDNA buffer (Gibco BRL), 2 μl of 0.1 x DTT (dithiothreitol, Gibco BRL) and 1 μl of 10 mM dNTP mix (Gibco BRL) and kept warm at 42°C for 2 minutes. The reaction mixture was added with 2 μg of reverse transcriptase (Superscript II RT) and allowed to react at 42°C for 1 hour.

The aforementioned reaction mixture was added with DEPC-treated water, 30 μl of 5 x 2nd strand reaction buffer, 3 μl of 10 mM dNTP, 1 μl of 10 U/μl DNA ligase, 4 μl of 10 U/μl DNA polymerase I and 2 U/μl of RNase H and allowed to react at 16°C for 2 hours. Then, the reaction mixture was added with 2 μl of 5 U/μl T4 DNA polymerase and allowed to react at 16°C for 5 minutes. The reaction mixture was added with 10 μl of 0.5 M EDTA. To this reaction mixture, an equal volume of a solution (phenol:chloroform = 1:1) was added, and these were mixed by vertically shaking the tube containing them. This mixture was centrifuged at 15000 rpm at 4°C for 10 minutes, and the aqueous layer was transferred to a new centrifuge tube. This aqueous layer was added with 1/10 volume of 3 M sodium acetate and 3-fold volume of 100% ethanol and mixed well. The mixture was left at -80°C for 10 minutes and then centrifuged at 15000 rpm at 4°C for 10 minutes. The precipitated pellet was rinsed twice with 70% ethanol, dried at room temperature for 5 minutes and then added with 12 μl of DEPC-treated water.

(ii) Synthesis of biotin-labeled cRNA probe

Subsequently, a biotin-labeled cRNA probe was synthesized from the double-stranded cDNA synthesized as described above by using Bio Array High Yield RNA Transcript Labeling Kit produced by Enzo. In a volume of 5 μl of double-stranded cDNA, 17 μl of DEPC-treated water, 4 μl of 10 x HY buffer, 4 μl of 10 x biotin-labeled ribonucleotide, 4 μl of 10 x DTT, 4 μl of 10 x RNase inhibitor mix and 2 μl of 20 x T7 RNA polymerase were mixed and allowed to react at 37°C for 4 hours.

Then, unreacted biotin-labeled ribonucleotides were removed from the solution of the biotin-labeled cRNA probe synthesized as described above by using RNeasy produced by Qiagen. The biotin-labeled cRNA probe solution was added with 160 μl of DEPC-treated water, mixed with 700 μl of RLT buffer, then added with 500 μl of 100% ethanol and mixed well. In a volume of 700 μl each of this solution was added to an RNeasy mini spin column and centrifuged at 8000 rpm for 15 seconds. The eluted solution was added to the RNeasy mini spin column again and centrifuged at 8000 rpm for 15 seconds. Subsequently, 500 μl of RPE buffer was added to the RNeasy mini spin column and centrifuged at 8000 rpm for 15 seconds. In a volume of 500 μl of RPE buffer was added to the RNeasy mini spin column again and centrifuged at 15000 rpm for 2 minutes.

The RNeasy mini spin column which was adsorbed with the biotin-labeled cRNA probe and washed as described above was transferred to a new centrifuge tube. In a volume of 30 μl of DEPC-treated water was added to the RNeasy mini spin column and left at room temperature for 1 minute. The mixture was centrifuged at 8000 rpm for 15 seconds to elute a purified biotin-labeled cRNA probe solution.

Subsequently, the purified biotin-labeled cRNA probe solution was fragmented. The biotin-labeled cRNA probe solution and 5 x fragmentation buffer (1/5 volume of the final solution volume was added) were mixed to obtain a biotin-labeled cRNA probe concentration of 0.5 μg/μl and allowed to react at 94°C for 35 minutes. The reaction mixture was subjected to 1% agarose gel electrophoresis to confirm that the probe had been cut into fragments having a length of about 100 bases.

(2) Hybridization

The fragmented biotin-labeled cRNA probe was first evaluated by using a test chip (TEST2 Chip) to confirm that the probe had no problem, and then the objective test of hybridization was performed. Rat chip sets (RG-U34A, RG-U34B, RG-U34C) were used for the objective test. These 3 types of rat chip sets include 7000 types of known rat genes and 17000 types of unknown genes in total. Hybridization was performed by the following procedure for the both cases using the test chip and the rat chip sets.

In an amount of 60 µg of the fragmented biotin-labeled cRNA probe, 12 µl of control oligonucleotide B2 (5 nM), 12 µl 100 x control cRNA cocktail, 12 µl of herring sperm DNA (10 mg/ml), 12 µl of acetylated BSA (50 mg/mg) and 600 µl of 2 x MES hybridization buffer were added, and the total volume was adjusted to 1200 µl with DEPC-treated water (referred to as "hybridization cocktail" hereinafter). The hybridization cocktail was heated at 99°C for 5 minutes to cause heat denaturation, left at 45°C for 5 minutes and centrifuged at 15000 rpm at room temperature for 5 minutes. For hybridization, the supernatant was added in a volume of 80 µl to the test chip (TEST2 Chip) or in a volume of 200 µl to each of the rat chip sets (RG-U34A, RG-U34B, RG-U34C).

The GeneChip was returned to room temperature, and prehybridization was performed with 1 x MES buffer (80 µl for the TEST2 chip and 200 µl for the rat chip sets) at 60 rpm at 45°C for 10 minutes. Subsequently, the prehybridization solution was removed, and the aforementioned heat-denatured hybridization cocktail was added. Hybridization was allowed at 45°C for 16 hours at 60 rpm.

(3) Washing, staining and scanning

The hybridization cocktail was removed from the GeneChip, and a non-stringent wash buffer was added to perform washing and staining by using Fluidic Station (Affymetrix). The washing and staining were performed according to the Mini_euk1 protocol of Fluidic Station for the TEST2 Chip and according to the EukGE-WS2 protocol for the rat chip sets. After the completion of the washing and staining, the chip was scanned with a scanner to obtain image data.

(4) Data analysis

Data analysis of the hybridization was performed by using GeneChip Analysis Suite. The expression level of each gene was represented by a relative value (average difference) based on the average value of expression levels of all the genes, which was taken as 100 (Table 1, (1) to (11)). In the table, the gene chip probe set names are identification numbers for management corresponding to the genes, which were designated by Affymetrix. UniGene is a database of DNA sequences registered at GenBank compiled according to gene (transcription product) types and species.

The column of 0hr in the table shows the gene expression levels in rat livers examined at 0 hour after partial hepatectomy. Similarly, the columns of 1hr, 6hr, 24hr, 48hr and 7d show gene expression levels in rat livers at 1, 6, 24, 48 hours and 7 days, respectively, after partial hepatectomy. The values in the columns of 0hr/0hr, 1hr/0hr, 6hr/0hr, 24hr/0hr, 48hr/0hr and 7d/0hr show relative values of gene expression levels at 0, 1, 6, 24, 48 hours and 7 days after partial hepatectomy based on the gene expression level at 0 hour after partial hepatectomy. The second decimal place of a value was rounded off. Further, values of expression levels of 0 or less were assumed as a value of 1 for convenience to obtain the expression ratio.

To produce a gene panel, known genes (genes of which sequence structures including all ORF had been registered in the GenBank database excluding those only of which partial sequences are known such as EST) were used.

For 77 types of known genes, expression levels in regenerating liver markedly increased as compared with the expression levels in normal liver (increased about 3 times or more, shown in boldfaces in Table 1). For 60 types of known genes, expression levels in regenerating liver markedly decreased as compared with the expression levels in normal liver (decreased to about 1/3 or less, shown in italics in Table 1). Among these, 7 types of known genes showed marked expression increases as well as decreases during liver regeneration. That is, marked expression changes (increases or decrease of 3 times or more from the expressions before hepatectomy) were observed during liver regeneration for 137 types of known genes.

Table 1 (1)

| No. | GeneChip probe set | Rat UniGene No | Gene |
|---|---|---|---|
| 1 | U17254_g_at | Rn. 10000 | immediate early gene transcription factor NGFI-B |
| 2 | J05460_s_at | Rn.10737 | P-450 cholesterol 7-alpha-hydroxylase |
| 3 | E01524cds_s_at | Rn.11359 | NADPH-cytochrome P450 redutase |
| 4 | AF030091 UTR#1_g_at | Rn.12962 | cyclin ania-6a |
| 5 | rc_AI1171231_at | Rn.16393 | amino acid transporter system A (ATA2) |
| 6 | rc_AI070318_at | Rn.17145 | connective tissue growth factor |
| 7 | M58587_at | Rn.1716 6 | Interieukin 6 receptor |

Table 1 (1) (continued)

| No. | GeneChip probe set | Rat UniGene No | Gene |
|---|---|---|---|
| 8 | AF036335_g_at | Rn.1926 | NonO/p54nrb homolog |
| 9 | D12769_g_at | Rn.19481 | BTE binding protein (same as 105-E9,126-A2) |
| 10 | rc_AI103604_at | Rn.19491 | deubiquitinating enzyme Ubp45 (ubp45) |
| 11 | rc_AA900505_at | Rn.2042 | rhoB |
| 12 | rc_AA849767_at | Rn.20670 | brain-enriched SH3-domain protein |
| 13 | rc_AI137820_at | Rn.22129 | CYR61 |
| 14 | rc_AA849718_g_at | Rn.2648 | interferon-inducible protein 16 |
| 15 | rc_AI101099_at | Rn.2714 | Metallothionein2 |
| 16 | M60921_at | Rn.27923 | B-cell translocation gene 2, anti-proliferative (PC3) |
| 17 | rc_AI008836_s_at | Rn.2874 | high mobility group protein 2 |
| 18 | S74351 s_ at | Rn.31120 | protein tyrosine phosphatase (dual specificity phosphate) |
| 19 | D37920_at | Rn.33239 | squalene epoxidase |
| 20 | M58634_at | Rn.34026 | Insulin-like growth factor binding protein 1 |
| 21 | rc_AI014163_at | Rn.3723 | interferon-related developmental regulator 1 (PC4) |
| 22 | AF020618_g_at | Rn.37483 | progression elevated gene 3 protein |
| 23 | rc_AA900348_s_at | Rn.40138 | tip associating protein (TAP) |
| 24 | rc AI072078_at | Rn.42905 | protein kinase KID2 (Kid2) |
| 25 | X54686cds_at | Rn.44317 7 | pJunB |
| 26 | J03190_at | Rn.6274 | 5-ar-rinotevulinate synthase |
| 27 | S77528cds_s_at | Rn.6479 | rNFIL-6=C/EBP-related transcription factor (C/EBP-beta) |
| 28 | AF023087_s_at | Rn.9096 | nerve growth factor induced factor A (Early growth response 1) |
| 29 | L27843_s_at | Rn.9459 | tyrosine phosphatase (PRL-1) |
| 30 | L41275cds_s_at | Rn.10089 | p21(WAF1) |
| 31 | rc_AI236828_s_at | Rn.10247 | Signal transducer and activator of transcription 3 |
| 32 | X68101_at | Rn.10431 | trg |
| 33 | M33962_g_at | Rn.11317 7 | Protein-tyrosine phosphatase |
| 34 | rc_AI234949_at | Rn.12262 | phocein protein |
| 35 | S85184_at | Rn.1294 | Cyclic Protein-2=cathepsin L proenzyme |
| 36 | 3AF063447_at | Rn.14550 | nuclear RNA helicase |
| 37 | M12919mRNA#2_at | Rn.1774 | Aldolase A fructose-bisphosphate |
| 38 | L23148_g_ at | Rn.2113 | Inhibitor of DNA binding 1, helix-loop-helix protein (splice variaton) |
| 39 | U21871_at | Rn.2143 | outer mitochondrial membrane receptor rTOM20 |
| 40 | X16038exon_s_at | Rn.25737 | alkaline phosphatase |
| 41 | X62952_at | Rn.271 0 | vimentin |
| 42 | M24067_at | Rn.29367 | Plasminogen activator inhibitor |

(continued)

| | | | |
|---|---|---|---|
| 43 | J02722cds_at | Rn.3160 | heme oxygenase |
| 44 | X58828_at | Rn.33497 | PTP-S mRNA for protein-tyrosine phosphatase |
| 45 | 016309_at | Rn.3483 | cyclin D3 |
| 46 | rc_AA892014_s_at | Rn.351 6 | liver nuclear protein p47 |
| 47 | U33500_g_at | Rn.37873 | retinol dehydrogenase type II |
| 48 | J00692_at | Rn.39438 | skeletal muscle alpha-actin |
| 49 | U75404UTR#1_s_at | Rn.42891 | Ssecks 322 (PKC binding protein) |
| 50 | U42627_at | Rn.431 3 | dual-specificity protein tyrosine phosphatase (rVH6) |
| 51 | rc_AA997933_at | Rn.43930 | Nopp140 associated protein (NAP65) |
| 52 | M14369exon#2_at | Rn.44576 | K-kininogen, differential splicing leads to HMW Kngk |
| 53 | Y14933mRNA_s_at | Rn.4881 2 | hepatocyte nuclear factor 6 beta |
| 54 | rc_AA900769_s_at | Rn.6321 | smooth muscle alpha-actin |
| 55 | rc_AI 146033_at | Rn.8459 | small zinc finger-like protein (TIM9a) |
| 56 | rc_AI176376_s_at | Rn.8925 | ATPase Na+/K+ transporting beta 1 polypeptide |
| 57 | rc_AI230712_at | Rn.950 | Subtilisin - like endoprotease |
| 58 | AB000717exons#1-8_s_at | Rn.9855 | non-hepatic-type S-adenosylmethionine synthetase |
| 59 | M24604_at | Rn.223 | proliferating cell nuclear antigen (PCNA/cyclin) mRNA |
| 60 | rc_AA860030_s_at | Rn.2458 | class I beta-tubulin |
| 61 | S80431_s_at | Rn.2571 6 | delta 4-3-ketosteroid 5 beta-reductase |
| 62 | J03786_s_at | Rn.2586 | Cytochrom P450 15-beta gene |
| 63 | rc_AI639082_s_at | Rn.33226 | mini chromosome maintenance deficient 6 |
| 64 | rc_AA818524_at | Rn.3611 5 | hnRNP protein, partial |
| 65 | rc_AA998683_g_at | Rn.3841 | Rat heat shock protein (Hsp27) |
| 66 | M33329 f at | Rn.40124 | hydroxysteroid sulfotransferase a (STa) |
| 67 | rc_AI231821 at | Rn.555 | Leukemia-associated cytosolic phosphoprotein stathmin |
| 68 | U64030_at | Rn.6102 | dUTPase |
| 69 | M22670cds_g_at | Rn.780 | alpha-2-macroglobulin |
| 70 | D14014_at | Rn.947 1 | cyclin D1 |
| 71 | L15079mRNA_s_at | Rn.9679 | P-glycoprotein 3/ multidrug resistance 2 |
| 72 | VO1227_s_at | Rn.3441 | alpha-tubulin |
| 73 | M37584_at | Rn.3636 | Rat histone (H2AZ) |
| 74 | rc_AA946368_at | Rn.3790 | FAT |
| 75 | rc_AI230970_f_at | Rn.11229 | Hemoglobin, alpha 1 |
| 76 | rc_AA925864_at | Rn.32702 | aldose-reductase-like protein MVDP/AKR1-B7 |
| 77 | D14989_f_at | Rn.40365 | hydroxysteroid sulfotransferase subunit |
| 78 | rc_AI234295_at | Rn.16933 | type IIb sodium-phosphate transporter |
| 79 | rc AI234100_f_at | Rn.2401 | cysteine rich protein |
| 80 | M11794cds#2_f_at | Rn.271 4 | Metallothionein 1(same as 6-E2) |

| | | | |
|---|---|---|---|
| 81 | J05210_at | Rn.29771 | ATP citrate lyase |
| 82 | AF089825_at | Rn.30020 | activin beta E |
| 83 | rc_AA943735_at | Rn.32110 | glycine transporter |
| 84 | X05684_at | Rn.48821 | Pyruvate kinase, liver and RBC |
| 85 | rc_AI009273_s_at | Rn.9486 | fatty acid synthase |
| 86 | M96601_at | Rn.9968 | taurine/beta-alanine transporter |
| 87 | L16995_at | | add1(helix-loop-helix protein associated with adipocyte determination and differentiation) |
| 88 | X91234_at | Rn. 10515 | 17-beta hydroxysteroid dehydrogenase type 2 |
| 89 | L37333_s_at | Rn.10992 | glucose-6-phosphatase (G6Pase) |
| 90 | D43964_at t | Rn.11129 | bile acid-Coenzyme A dehydrogenase: amino acid n-acyltransferase |
| 91 | X74593_at | Rn.11334 | Sorbitol dehydrogenase |
| 92 | rc_AA852046_s_at | Rn.11350 | VL30 element (same as 12-C10) |
| 93 | rc_ AA892553_at | Rn.12592 | signal transducer and activator of transcription (Stat1) |
| 94 | rc_AI113166_s_at | Rn.15739 | Apolipoprotein C-IV |
| 95 | rc_ AA818627_at | Rn.16736 | ISI1 RAT INSULIN-INDUCED PROTEIN 1 |
| 96 | AB016800_g_at | Rn.228 | 7-dehydrocholesterol reductase |
| 97 | rc_AI233209_ at | Rn.24561 | CDK108 |
| 98 | rc_AA893485_at | Rn.31772 | Glu-Pro dipeptide repeat protein |
| 99 | M95591_at | Rn.3252 | famesyl diphosphate farnesyl transferase 1 |
| 100 | L25785_at | Rn.3545 | Transforming growth factor beta stimulated clone 22 |
| 101 | D17370_at | Rn.3881 | cystathionine gamma-lyase |
| 102 | AJ011656cds s at | Rn.451 3 | claudin 3 |
| 103 | rc AI177004_s_at | Rn.5106 | 3-hydroxy-3-methylglutaryl-Coenzyme A synthase 1 |
| 104 | rc AI172293 at | Rn.716 7 | RANP-1 |
| 105 | rc AA957498_at | Rn.8548 | androgen-inducible aldehyde reductase |
| 106 | rc_AI232087_at | Rn.A 10417 7 | (S)-2-hydroxy acid oxidase |
| 107 | M18363cds_s_ at | Rn.10870 | Cytochrome P450, subfamily IIC (mephenytoin 4-hydroxylase) |
| 108 | J02585_at | Rn.10982 | liver stearyl-CoA desaturase |
| 109 | rc_AI044610_s_at | Rn.11064 | Dopa decarboxylase (aromatic L-amino acid decarboxylase) |
| 110 | X06150cds. g_at | Rn.11142 | glycine methyltransferase |
| 111 | J05031 _g_at | Rn.147 | Isovaleryl Coenzyme A dehydrogenase |
| 112 | rc_AA925393_at | Rn.1551 6 | acetyl-coenzyme A carboxylase |
| 113 | rc_AA926200_at | Rn.1568 1 | aldehyde oxidase (female form) |
| 114 | AF037072_at | Rn.1647 | carbonic anhydrase 3 |
| 115 | rc AA819899_at | Rn.1829 | transcobalamin II precursor (TCII) |
| 116 | M22359mRNA s_at | Rn.1911 1 | plasma proteinase inhibitor alpha-1-inhibitor III |
| 117 | K00996rnRNA_s_at | Rn.2287 | cytochrome p-450e (2b19(Cyp2b15)) |
| 118 | M59861_at | Rn.2328 | 10-formyltetrahydrofolate dehydrogenase |

(continued)

| | | | |
|---|---|---|---|
| 119 | rc_AI169656_at | Rn.23348 | organic anion transporter 3 |
| 120 | X14552 at | Rn.33965 | Caldesmon 1 |

| | | | |
|---|---|---|---|
| 121 | Y07534cds_s_at | Rn34396 | Serine protease inhibitor (cytochrome P451 c27/25) |
| 122 | J02827_g_at | Rn3489 | Branched chain alpha-ketoacid dehydrogenase subunit E1 alpha |
| 123 | K03249_at | Rn3671 | peroxisomal enoyl-CoA- hydrotase-3-trycroxyacyl-CoA bifunctional enzyme |
| 124 | rc_AI639418_at | Fh42914 | type I thyroxine deiodinase |
| 125 | U88036_at | Rn5641 | brain digoxin carrier protein |
| 126 | X65296cds_s_at | Rn6066 | carboxylesterase (Es-HVEL (cholesterol esterase) |
| 127 | D10354_s_at | Rn6318 | alanine aminotransferase |
| 128 | rc_AI144586_at | Rn8415 | evectin-1 (EVT1) |
| 129 | M84719_ at | Rn867 | flavin-containing monooxygenase 1 (FMO-1) |
| 130 | K01934mRN4#2_at | Rn9854 | Thyroid hormone responsive protein (spot14) |
| 131 131 | M77479 at M77479_at | Rn9913 Rn9913 | Solute carrier family 10 (sodium/bile acid cotransporter family), member 1 |
| 132 | J03863_at | Rn9918 | serine dehydratase (SDH2) |
| 133 | L22339_g_at | Rn9937 | N-hydroxy-2-acetylaminofluorene (ST1C1) |
| 134 | X79081 mRNA_f_at | | p450CYP2C11 gene for cytochrome |
| 135 | rc_AA945418_at | Rn23013 | sterol 12alpha-hydroxylase P450 |
| 136 | rc_AI231076_at | Rn3572 | IBP4_RAT INSULIN-LIKE CROWTH FACTOR BINDING PROTEIN 4 PRECURSOR |
| 137 | U10697_s_at | Rn10050 | kidney microsomal carboxylesterase |

Table 1 (5)

| | | Gene expression intensity | | | | | | Ratio of expression level after hepatectomy to expression level before hepatectomy (0hr) (GeneChip) | | | | | | | Ratio of expression level after hepatectomy to expression level before hepatectomy (0hr) (ATAC) | | | | | | | Information about primers for ATAC-PCR | |
|---|---|---|---|---|---|---|---|---|---|---|---|---|---|---|---|---|---|---|---|---|---|---|---|
| No. | GeneChip probe set | 0hr | 1hr | 6hr | 24hr | 48hr | 7d | 0hr | 1hr | 6hr | 24hr | 48hr | 7d | Group | 0hr | 1hr | 6hr | 24hr | 48hr | 7d | Correlation with GeneChip data | SEQ ID NO. | Used restriction enzyme (MboI unless otherwise specified) |
| 1 | U17254_g_at | 26 | 765 | 112 | 6 | 60 | 32 | 1 | 29 | 4.3 | *0.2* | 2.3 | 1.2 | 1 | 1.0 | 14.9 | 1.3 | 1.3 | 3.2 | 0.8 | 0.985 | 1 | |
| 2 | J05460_s_at | 168 | 1042 | 1 | 180 | 157 | 636 | 1 | 6.2 | *0* | 1.1 | 0.9 | 3.8 | 1 | | | | | | | | 2 | |
| 3 | E01524cds_s_at | 111 | 530 | 596 | 147 | 95 | 124 | 1 | 4.8 | 5.4 | 1.3 | 0.9 | 1.1 | 1 | 1.0 | 6.4 | 7.1 | 2.6 | 2.3 | 1.8 | 0.982 | 3 | |
| 4 | AF030091UTR#1_g_at | 39 | 461 | 533 | 171 | 275 | 275 | 1 | 12 | 14 | 4.4 | 7.1 | 7.1 | 1 | | | | | | | | 4 | |
| 5 | rc_AI171231_at | 94 | 966 | 1330 | 92 | 129 | 218 | 1 | 10 | 14 | 1 | 1.4 | 2.3 | 1 | 1.0 | 11.2 | 13.1 | 3.5 | 1.2 | 1.8 | 0.975 | 5 | |
| 6 | rc_AI070318_at | 53 | 202 | 437 | 118 | 99 | 88 | 1 | 3.8 | 8.2 | 2.2 | 1.9 | 1.7 | 1 | 1.0 | 4.3 | 4.1 | 8.4 | 3.0 | 1.3 | 0.225 | 6 | |
| 7 | M58587_at | 86 | 343 | 416 | 69 | 94 | 114 | 1 | 4 | 4.8 | 0.8 | 1.1 | 1.3 | 1 | 1.0 | 1.5 | 1.5 | 1.0 | 0.9 | 1.3 | 0.875 | 7 | |
| 8 | AF036335_g_at | 12 | 36 | 114 | 56 | 54 | 37 | 1 | 3 | 9.5 | 4.7 | 4.5 | 3.1 | 1 | 1.0 | 140.5 | 676.2 | 261.9 | 258.2 | 311.3 | 0.958 | 8 | |
| 9 | D12769_g_at | 189 | 713 | 482 | 118 | 220 | 259 | 1 | 3.8 | 2.6 | 0.6 | 1.2 | 1.4 | 1 | 1.0 | 2.2 | 1.3 | 0.8 | 0.4 | 1.5 | 0.814 | 9 | |
| 10 | rc_AI103604_at | 166 | 978 | 99 | 279 | 267 | 237 | 1 | 5.9 | 0.6 | 1.7 | 1.6 | 1.4 | 1 | 1.0 | 0.3 | 0.9 | 1.0 | 0.9 | 0.5 | -0.787 | 10 | |
| 11 | rc_AA900505_at | 28 | 222 | 133 | 91 | 193 | 83 | 1 | 7.9 | 4.8 | 3.3 | 6.9 | 3 | 1 | 1.0 | 4.4 | 2.0 | 2.1 | 4.1 | 5.0 | 0.579 | 11 | |
| 12 | rc_AA849767_at | 71 | 589 | 328 | 307 | 161 | 145 | 1 | 8.3 | 4.6 | 4.3 | 2.3 | 2 | 1 | | | | | | | | 12 | *Nla* III |
| 13 | rc_AI137820_at | 44 | 244 | 103 | 71 | 91 | 58 | 1 | 5.5 | 2.3 | 1.6 | 2.1 | 1.3 | 1 | | | | | | | | 13 | |
| 14 | rc_AA849718_g_at | 162 | 705 | 612 | 586 | 558 | 964 | 1 | 4.4 | 3.8 | 3.6 | 3.4 | 6 | 1 | | | | | | | | 14 | |
| 15 | rc_AI101099_at | 264 | 1421 | 1374 | 236 | 137 | 502 | 1 | 5.4 | 5.2 | 0.9 | 0.5 | 1.9 | 1 | | | | | | | | 15 | |
| 16 | M60921_at | 155 | 547 | 566 | 257 | 221 | 270 | 1 | 3.5 | 3.7 | 1.7 | 1.4 | 1.7 | 1 | 1.0 | 18.4 | 16.8 | 3.5 | 4.0 | 3.8 | 0.984 | 16 | |
| 17 | rc_AI008836_s_at | 4 | 40 | 33 | 166 | 245 | 112 | 1 | 10 | 8.3 | 42 | 61 | 28 | 1 | 1.0 | *0.3* | *0.2* | 1.0 | 1.6 | 2.2 | 0.562 | 17 | |
| 18 | S74351_s_at | 159 | 667 | 165 | 69 | 74 | 15 | 1 | 4.2 | 1 | 0.4 | 0.5 | *0.1* | 1 | 1.0 | 5.8 | 1.1 | 0.3 | 1.0 | 3.8 | 0.730 | 18 | |
| 19 | D37920_at | 17 | 57 | 124 | 36 | 28 | 97 | 1 | 3.4 | 7.3 | 2.1 | 1.6 | 5.7 | 1 | 1.0 | 3.7 | 4.4 | 4.4 | 4.0 | 3.8 | 0.516 | 19 | |
| 20 | M58634_at | 100 | 986 | 924 | 37 | 1 | 241 | 1 | 9.9 | 9.2 | 0.4 | *0* | 2.4 | 1 | 1.0 | 20.9 | 9.5 | 1.2 | 3.6 | 5.7 | 0.882 | 20 | |

Table 1 (6)

| No. | GeneChip probe set | Gene expression intensity | | | | | | Ratio of expression level after hepatectomy to expression level before hepatectomy (0hr) (GeneChip) | | | | | | Group | Ratio of expression level after hepatectomy to expression level before hepatectomy (0hr) (ATAC) | | | | | | | Information about primers for ATAC-PCR | |
|---|---|---|---|---|---|---|---|---|---|---|---|---|---|---|---|---|---|---|---|---|---|---|---|
| | | 0hr | 1hr | 6hr | 24hr | 48hr | 7d | 0hr | 1hr | 6hr | 24hr | 48hr | 7d | | 0hr | 1hr | 6hr | 24hr | 48hr | 7d | Correlation with GeneChip data | SEQ ID NO. | Used restriction enzyme (MboI unless otherwise specified) |
| 21 | rc_AI014163_at | 10 | 297 | 143 | 27 | 49 | 50 | 1 | 30 | 14 | 2.7 | 4.9 | 5 | 1 | 1.0 | 1.7 | 1.0 | 0.7 | 0.6 | 0.3 | 0.818 | 21 | |
| 22 | AF020618_g_at | 40 | 333 | 60 | 66 | 66 | 25 | 1 | 8.3 | 1.5 | 1.7 | 1.7 | 0.6 | 1 | 1.0 | 1.3 | 0.9 | 0.4 | 0.9 | 0.4 | 0.693 | 22 | |
| 23 | rc_AA900348_s_at | 40 | 286 | 346 | 274 | 243 | 238 | 1 | 7.2 | 8.7 | 6.9 | 6.1 | 6 | 1 | 1.0 | 5.4 | 3.4 | 2.3 | 5.5 | 4.9 | 0.567 | 23 | |
| 24 | rc_AI072078_at | 86 | 378 | 122 | 34 | 70 | 70 | 1 | 4.4 | 1.4 | 0.4 | 0.8 | 0.8 | 1 | 1.0 | 7.1 | 1.4 | 0.6 | 0.9 | 1.0 | 0.992 | 24 | |
| 25 | X54686cds_at | 52 | 240 | 195 | 21 | 38 | 53 | 1 | 4.6 | 3.8 | 0.4 | 0.7 | 1 | 1 | | | | | | | | 25 | |
| 26 | J03190_at | 255 | 1051 | 870 | 377 | 315 | 189 | 1 | 4.1 | 3.4 | 1.5 | 1.2 | 0.7 | 1 | 1.0 | 5.6 | 2.4 | 1.2 | 2.2 | 0.9 | 0.868 | 26 | |
| 27 | S77528cds_s_at | 143 | 498 | 364 | 181 | 208 | 158 | 1 | 3.5 | 2.5 | 1.3 | 1.5 | 1.1 | 1 | 1.0 | 4.4 | 3.3 | 1.7 | 1.9 | 1.7 | 0.988 | 27 | |
| 28 | AF023087_s_at | 107 | 1362 | 961 | 187 | 254 | 616 | 1 | 13 | 9 | 1.7 | 2.4 | 5.8 | 1 | 1.0 | 1.8 | 1.8 | 1.5 | 2.4 | 2.4 | 0.273 | 28 | |
| 29 | L27843_s_at | 226 | 1061 | 1033 | 459 | 482 | 324 | 1 | 4.7 | 4.6 | 2 | 2.1 | 1.4 | 1 | 1 | 10.4 | 10.5 | 6.1 | 3.5 | 26.8 | 0.023 | 29 | |
| 30 | L41275cds_s_at | 44 | 18 | 235 | 98 | 126 | 20 | 1 | 0.4 | 5.3 | 2.2 | 2.9 | 0.5 | 2 | | | | | | | | 30 | |
| 31 | rc_AI236828_s_at | 240 | 298 | 1128 | 249 | 176 | 745 | 1 | 1.2 | 4.7 | 1 | 0.7 | 3.1 | 2 | | | | | | | | 31 | |
| 32 | X68101_at | 11 | 28 | 49 | 69 | 117 | 55 | 1 | 2.5 | 4.5 | 6.3 | 11 | 5 | 2 | | | | | | | | 32 | |
| 33 | M33962_g_at | 128 | 166 | 471 | 154 | 139 | 138 | 1 | 1.3 | 3.7 | 1.2 | 1.1 | 1.1 | 2 | | | | | | | | 33 | |
| 34 | rc_AI234949_at | 42 | 44 | 322 | 79 | 85 | 33 | 1 | 1 | 7.7 | 1.9 | 2 | 0.8 | 2 | 1.0 | 1.7 | 2.7 | 1.9 | 0.5 | 0.6 | 0.751 | 34 | |
| 35 | S85184_at | 92 | 221 | 393 | 144 | 132 | 125 | 1 | 2.4 | 4.3 | 1.6 | 1.4 | 1.4 | 2 | 1.0 | 2.3 | 4.1 | 3.1 | 1.6 | 3.4 | 0.691 | 35 | |
| 36 | AF063447_at | 22 | 22 | 70 | 124 | 118 | 73 | 1 | 1 | 3.2 | 5.6 | 5.4 | 3.3 | 2 | 1.0 | 3.2 | 2.2 | 3.1 | 1.8 | 1.1 | 0.154 | 36 | |
| 37 | M12919mRNA#2_at | 79 | 97 | 359 | 185 | 151 | 84 | 1 | 1.2 | 4.5 | 2.3 | 1.9 | 1.1 | 2 | | | | | | | | 37 | |
| 38 | L23148_g_at | 19 | 44 | 134 | 153 | 79 | 49 | 1 | 2.3 | 7.1 | 8.1 | 4.2 | 2.6 | 2 | 1.0 | 2.6 | 5.3 | 3.7 | 4.5 | 1.0 | 0.781 | 38 | |
| 39 | U21871_at | 195 | 265 | 768 | 432 | 502 | 338 | 1 | 1.4 | 3.9 | 2.2 | 2.6 | 1.7 | 2 | | | | | | | | 39 | |
| 40 | X16038exon_s_at | 31 | 51 | 354 | 75 | 49 | 51 | 1 | 1.6 | 11 | 2.4 | 1.6 | 1.6 | 2 | | | | | | | | 40 | |

Table 1 (7)

| | | Gene expression intensity | | | | | | Ratio of expression level after hepatectomy to expression level before hepatectomy (0hr) (GeneChip) | | | | | | | Ratio of expression level after hepatectomy to expression level before hepatectomy (0hr) (ATAC) | | | | | | | Information about primers for ATAC-PCR | |
|---|---|---|---|---|---|---|---|---|---|---|---|---|---|---|---|---|---|---|---|---|---|---|---|
| No. | GeneChip probe set | 0hr | 1hr | 6hr | 24hr | 48hr | 7d | 0hr | 1hr | 6hr | 24hr | 48hr | 7d | Group | 0hr | 1hr | 6hr | 24hr | 48hr | 7d | Correlation with GeneChip data | SEQ ID NO. | Used restriction enzyme (MboI unless otherwise specified) |
| 41 | X62952_at | 29 | 58 | 95 | 176 | 189 | 93 | 1 | 2 | 3.3 | 6.1 | 6.5 | 3.2 | 2 | 1.0 | 3.7 | 9.4 | 7.9 | 10.7 | 8.1 | 0.808 | 41 | |
| 42 | M24067_at | 31 | 69 | 210 | 59 | 48 | 45 | 1 | 2.2 | 6.8 | 1.9 | 1.5 | 1.5 | 2 | | | | | | | | 42 | |
| 43 | J02722cds_at | 18 | 31 | 762 | 57 | 36 | 57 | 1 | 1.7 | 42 | 3.2 | 2 | 3.2 | 2 | 1.0 | 0.6 | 4.2 | 2.2 | 1.9 | 0.8 | 0.887 | 43 | |
| 44 | X58828_at | 7 | 12 | 124 | 19 | 58 | 22 | 1 | 1.7 | 18 | 2.7 | 8.3 | 3.1 | 2 | | | | | | | | 44 | |
| 45 | D16309_at | 32 | 32 | 180 | 95 | 79 | 48 | 1 | 1 | 5.6 | 3 | 2.5 | 1.5 | 2 | | | | | | | | 45 | |
| 46 | rc_AA892014_s_at | 43 | 58 | 233 | 110 | 185 | 137 | 1 | 1.3 | 5.4 | 2.6 | 4.3 | 3.2 | 2 | | | | | | | | 46 | |
| 47 | U33500_g_at | 107 | 286 | 374 | 125 | 124 | 14 | 1 | 2.7 | 3.5 | 1.2 | 1.2 | *0.1* | 2 | 1.0 | 1.9 | 6.0 | 1.0 | 1.0 | 1.2 | 0.822 | 47 | |
| 48 | J00692_at | 9 | 7 | 154 | 9 | 4 | 11 | 1 | 0.8 | 17 | 1 | 0.4 | 1.2 | 2 | | | | | | | | 48 | |
| 49 | U75404UTR#1_s_at | 23 | 31 | 141 | 36 | 38 | 28 | 1 | 1.3 | 6.1 | 1.6 | 1.7 | 1.2 | 2 | | | | | | | | 49 | *Bfa* I |
| 50 | U42627_at | 25 | 51 | 103 | 63 | 104 | 45 | 1 | 2 | 4.1 | 2.5 | 4.2 | 1.8 | 2 | 1.0 | 2.0 | 2.8 | 1.7 | 3.8 | 6.1 | 0.219 | 50 | |
| 51 | rc_AA997933_at | 73 | 52 | 592 | 214 | 178 | 163 | 1 | 0.7 | 8.1 | 2.9 | 2.4 | 2.2 | 2 | | | | | | | | 51 | |
| 52 | M14369exon#2_at | 39 | 40 | 385 | 17 | 34 | 105 | 1 | 1 | 9.9 | 0.4 | 0.9 | 2.7 | 2 | | | | | | | | | |
| 53 | Y14933mRNA_s_at | 19 | 9 | 184 | 27 | 190 | 131 | 1 | 0.5 | 9.7 | 1.4 | 10 | 6.9 | 2 | 1 | 1.8 | 3.5 | 1.3 | 1.7 | 2.1 | 0.662 | 52 | |
| 54 | rc_AA900769_s_at | 22 | 12 | 80 | 122 | 176 | 34 | 1 | 0.5 | 3.6 | 5.5 | 8 | 1.5 | 2 | 1.0 | 0.9 | 6.0 | 4.0 | 6.0 | 2.6 | 0.829 | 53 | |
| 55 | rc_AI146033_at | 159 | 151 | 701 | 281 | 281 | 180 | 1 | 0.9 | 4.4 | 1.8 | 1.8 | 1.1 | 2 | | | | | | | | 54 | *Hpa* II |
| 56 | rc_AI176376_s_at | 144 | 343 | 1274 | 335 | 283 | 147 | 1 | 2.4 | 8.8 | 2.3 | 2 | 1 | 2 | | | | | | | | 55 | |
| 57 | rc_AI230712_at | 29 | 26 | 169 | 9 | 27 | 25 | 1 | 0.9 | 5.8 | *0.3* | 0.9 | 0.9 | 2 | | | | | | | | 56 | |
| 58 | AB000717exons#1-8_s_at | 16 | 34 | 114 | 48 | 47 | 40 | 1 | 2.1 | 7.1 | 3 | 2.9 | 2.5 | 2 | | | | | | | | | |
| 59 | M24604_at | 118 | 184 | 257 | 820 | 442 | 224 | 1 | 1.6 | 2.2 | 6.9 | 3.7 | 1.9 | 3 | | | | | | | | | |
| 60 | rc_AA860030_s_at | 193 | 216 | 236 | 1119 | 699 | 463 | 1 | 1.1 | 1.2 | 5.8 | 3.6 | 2.4 | 3 | | | | | | | | 57 | |

Table 1 (8)

| | | Gene expression intensity | | | | | | Ratio of expression level after hepatectomy to expression level before hepatectomy (0hr) (GeneChip) | | | | | | | Ratio of expression level after hepatectomy to expression level before hepatectomy (0hr) (ATAC) | | | | | | | Information about primers for ATAC-PCR | |
|---|---|---|---|---|---|---|---|---|---|---|---|---|---|---|---|---|---|---|---|---|---|---|---|
| No. | GeneChip probe set | 0hr | 1hr | 6hr | 24hr | 48hr | 7d | 0hr | 1hr | 6hr | 24hr | 48hr | 7d | Group | 0hr | 1hr | 6hr | 24hr | 48hr | 7d | Correlation with GeneChip data | SEQ ID NO. | Used restriction enzyme (MboI unless otherwise specified) |
| 61 | S80431_s_at | 36 | 66 | 17 | 285 | 356 | 208 | 1 | 1.8 | 0.5 | 7.9 | 9.9 | 5.8 | 3 | | | | | | | | 58 | *Bfa* I |
| 62 | J03786_s_at | 84 | 183 | 162 | 416 | 555 | 170 | 1 | 2.2 | 1.9 | 5 | 6.6 | 2 | 3 | 1.0 | 3.5 | 5.8 | 6.6 | 12.3 | 7.2 | 0.841 | 59 | |
| 63 | rc_AI639082_s_at | 41 | 36 | 27 | 191 | 100 | 76 | 1 | 0.9 | 0.7 | 4.7 | 2.4 | 1.9 | 3 | 1.0 | 0.7 | 1.1 | 0.7 | 3.0 | 2.7 | 0.027 | 60 | |
| 64 | rc_AA818524_at | 21 | 26 | 57 | 234 | 197 | 267 | 1 | 1.2 | 2.7 | 11 | 9.4 | 13 | 3 | 1.0 | 1.7 | 1.9 | 2.6 | 2.5 | 4.7 | 0.852 | 61 | |
| 65 | rc_AA998683_g_at | 73 | 181 | 65 | 394 | 259 | 37 | 1 | 2.5 | 0.9 | 5.4 | 3.5 | 0.5 | 3 | 1.0 | 0.9 | 0.7 | 1.7 | 3.9 | 1.2 | 0.516 | 62 | |
| 66 | M33329_f_at | 180 | 406 | 134 | 633 | 633 | 896 | 1 | 2.3 | 0.7 | 3.5 | 3.5 | 5 | 3 | 1.0 | 1.5 | 0.4 | 0.9 | 1.3 | 2.6 | 0.795 | 63 | |
| 67 | rc_AI231821_at | 37 | 56 | 13 | 227 | 385 | 179 | 1 | 1.5 | 0.4 | 6.1 | 10 | 4.8 | 3 | 1.0 | 1.4 | 0.4 | 4.6 | 1.1 | 0.7 | 0.308 | 64 | |
| 68 | U64030_at | 29 | 38 | 28 | 207 | 233 | 97 | 1 | 1.3 | 1 | 7.1 | 8 | 3.3 | 3 | 1 | 0.4 | *0.3* | 0.9 | 0.6 | 0.4 | 0.270 | 65 | |
| 69 | M22670cds_g_at | 64 | 71 | 105 | 210 | 54 | 47 | 1 | 1.1 | 1.6 | 3.3 | 0.8 | 0.7 | 3 | | | | | | | | 66 | *Nla* III |
| 70 | D14014_at | 180 | 189 | 79 | 713 | 553 | 261 | 1 | 1.1 | 0.4 | 4 | 3.1 | 1.5 | 3 | 1.0 | 1.6 | 2.5 | 1.5 | 3.9 | 0.8 | 0.258 | 67 | |
| 71 | L15079mRNA_s_at | 129 | 144 | 69 | 513 | 486 | 184 | 1 | 1.1 | 0.5 | 4 | 3.8 | 1.4 | 3 | 1 | 2.3 | 1.1 | 3.5 | 1.9 | 1.4 | 0.740 | 68 | |
| 72 | V01227_s_at | 48 | 59 | 79 | 135 | 156 | 97 | 1 | 1.2 | 1.6 | 2.8 | 3.3 | 2 | 4 | | | | | | | | 69 | |
| 73 | M37584_at | 173 | 156 | 162 | 447 | 693 | 450 | 1 | 0.9 | 0.9 | 2.6 | 4 | 2.6 | 4 | | | | | | | | 70 | *Bfa* I |
| 74 | rc_AA946368_at | 34 | 45 | 79 | 86 | 157 | 191 | 1 | 1.3 | 2.3 | 2.5 | 4.6 | 5.6 | 4 | | | | | | | | 71 | |
| 75 | rc_AI230970_f_at | 462 | 1181 | 856 | 1227 | 1031 | 1606 | 1 | 2.6 | 1.9 | 2.7 | 2.2 | 3.5 | 5 | 1.0 | 1.4 | 1.1 | 0.9 | 0.7 | 2.6 | 0.678 | 72 | |
| 76 | rc_AA925864_at | 22 | 4 | 50 | 55 | 64 | 358 | 1 | *0.2* | 2.3 | 2.5 | 2.9 | 16 | 5 | 1.0 | 1.4 | 1.5 | 2.7 | 3.3 | 2.7 | 0.458 | 73 | |
| 77 | D14989_f_at | 147 | 296 | 101 | 414 | 334 | 586 | 1 | 2 | 0.7 | 2.8 | 2.3 | 4 | 5 | | | | | | | | 74 | *Nla* III |
| 78 | rc_AI234295_at | 180 | 44 | 18 | 282 | 500 | 226 | 1 | *0.2* | *0.1* | 1.6 | 2.8 | 1.3 | 6 | | | | | | | | 75 | |
| 79 | rc_AI234100_f_at | 240 | 68 | 17 | 148 | 256 | 237 | 1 | *0.3* | *0.1* | 0.6 | 1.1 | 1 | 6 | 1.0 | 2.6 | 2.8 | 0.8 | 0.8 | 0.9 | -0.883 | 76 | |
| 80 | M11794cds#2_f_at | 1181 | 289 | 1 | 902 | 593 | 912 | 1 | *0.2* | *0* | 0.8 | 0.5 | 0.8 | 6 | 1.0 | 5.3 | 19.8 | 2.6 | 1.2 | 3.9 | -0.775 | 77 | |

Table 1 (9)

| | | Gene expression intensity | | | | | | Ratio of expression level after hepatectomy to expression level before hepatectomy (0hr) (GeneChip) | | | | | | | Ratio of expression level after hepatectomy to expression level before hepatectomy (0hr) (ATAC) | | | | | | | Information about primers for ATAC-PCR | |
|---|---|---|---|---|---|---|---|---|---|---|---|---|---|---|---|---|---|---|---|---|---|---|---|
| No. | GeneChip probe set | 0hr | 1hr | 6hr | 24hr | 48hr | 7d | 0hr | 1hr | 6hr | 24hr | 48hr | 7d | Group | 0hr | 1hr | 6hr | 24hr | 48hr | 7d | Correlation with GeneChip data | SEQ ID NO. | Used restriction enzyme (MboI unless otherwise specified) |
| 81 | J05210_at | 533 | 124 | 112 | 61 | 110 | 446 | 1 | *0.2* | *0.2* | *0.1* | *0.2* | 0.8 | 6 | 1.0 | 0.9 | *0.1* | 0.4 | *0.1* | *0.1* | 0.314 | 78 | |
| 82 | AF089825_at | 246 | 39 | 52 | 36 | 63 | 47 | 1 | *0.2* | *0.2* | *0.1* | *0.3* | *0.2* | 6 | | | | | | | | 79 | |
| 83 | rc_AA943735_at | 643 | 167 | 20 | 1 | 11 | 38 | 1 | *0.3* | *0* | *0* | *0* | *0.1* | 6 | | | | | | | | | |
| 84 | X05684_at | 214 | 35 | 107 | 24 | 22 | 100 | 1 | *0.2* | 0.5 | *0.1* | *0.1* | 0.5 | 6 | | | | | | | | 80 | |
| 85 | rc_AI009273_s_at | 1721 | 369 | 513 | 313 | 262 | 2000 | 1 | *0.2* | *0.3* | *0.2* | *0.2* | 1.2 | 6 | 1.0 | 1.3 | 0.7 | 0.4 | 0.9 | 3.1 | 0.736 | 81 | |
| 86 | M96601_at | 168 | 23 | 23 | 91 | 200 | 55 | 1 | *0.1* | *0.1* | 0.5 | 1.2 | *0.3* | 6 | 1 | 1.9 | 0.6 | 1.0 | 0.3 | 0.7 | -0.459 | 82 | |
| 87 | L16995_at | 386 | 111 | 51 | 25 | 174 | 217 | 1 | *0.3* | *0.1* | *0.1* | 0.5 | 0.6 | 6 | | | | | | | | 83 | |
| 88 | X91234_at | 464 | 887 | 1 | 1 | 22 | 406 | 1 | 1.9 | *0* | *0* | *0* | 0.9 | 7 | 1.0 | 2.5 | *0.1* | *0.1* | *0.0* | *0.1* | 0.892 | 84 | |
| 89 | L37333_s_at | 674 | 592 | 102 | 229 | 162 | 577 | 1 | 0.9 | *0.2* | *0.3* | *0.2* | 0.9 | 7 | 1.0 | 2.0 | 0.4 | *0.3* | 0.4 | 0.4 | 0.609 | 85 | |
| 90 | D43964_at | 966 | 832 | 218 | 749 | 699 | 707 | 1 | 0.9 | *0.2* | 0.8 | 0.7 | 0.7 | 7 | | | | | | | | 86 | *Bfa* I |
| 91 | X74593_at | 759 | 688 | 176 | 428 | 462 | 526 | 1 | 0.9 | *0.2* | 0.6 | 0.6 | 0.7 | 7 | 1.0 | 0.7 | 0.9 | *0.3* | 1.8 | 2.6 | 0.013 | 87 | |
| 92 | rc_AA852046_s_at | 1524 | 1401 | 336 | 1430 | 1187 | 1292 | 1 | 0.9 | *0.2* | 0.9 | 0.8 | 0.8 | 7 | | | | | | | | 88 | |
| 93 | rc_AA892553_at | 55 | 58 | 1 | 17 | 143 | 58 | 1 | 1.1 | *0* | *0.3* | 2.6 | 1.1 | 7 | | | | | | | | 89 | *Nla* III |
| 94 | rc_AI113166_s_at | 1549 | 941 | 480 | 436 | 177 | 283 | 1 | 0.6 | *0.3* | *0.3* | *0.1* | *0.2* | 7 | 1.0 | 1.7 | 1.2 | 0.5 | 0.4 | 0.7 | 0.517 | 90 | |
| 95 | rc_AA818627_at | 743 | 472 | 203 | 688 | 565 | 252 | 1 | 0.6 | *0.3* | 0.9 | 0.8 | *0.3* | 7 | 1.0 | 0.8 | 0.5 | 0.9 | 0.4 | 0.4 | 0.708 | 91 | |
| 96 | AB016800_g_at | 340 | 175 | 55 | 279 | 214 | 319 | 1 | 0.5 | *0.2* | 0.8 | 0.6 | 0.9 | 7 | 1.0 | 1.1 | 0.6 | 1.6 | 1.6 | 3.6 | 0.529 | 92 | |
| 97 | rc_AI233209_at | 138 | 142 | 43 | 33 | 82 | 15 | 1 | 1 | *0.3* | *0.2* | 0.6 | *0.1* | 7 | | | | | | | | 93 | |
| 98 | rc_AA893485_at | 179 | 159 | 12 | 59 | 117 | 110 | 1 | 0.9 | *0.1* | *0.3* | 0.7 | 0.6 | 7 | | | | | | | | 94 | |
| 99 | M95591_at | 155 | 102 | 1 | 16 | 77 | 235 | 1 | 0.7 | *0* | *0.1* | 0.5 | 1.5 | 7 | | | | | | | | 95 | |
| 100 | L25785_at | 801 | 559 | 112 | 344 | 264 | 356 | 1 | 0.7 | *0.1* | 0.4 | *0.3* | 0.4 | 7 | | | | | | | | 96 | |

Table 1 (10)

| | | Gene expression intensity | | | | | | Ratio of expression level after hepatectomy to expression level before hepatectomy (0hr) (GeneChip) | | | | | | | Ratio of expression level after hepatectomy to expression level before hepatectomy (0hr) (ATAC) | | | | | | | Information about primers for ATAC-PCR | |
|---|---|---|---|---|---|---|---|---|---|---|---|---|---|---|---|---|---|---|---|---|---|---|---|
| No. | GeneChip probe set | 0hr | 1hr | 6hr | 24hr | 48hr | 7d | 0hr | 1hr | 6hr | 24hr | 48hr | 7d | Group | 0hr | 1hr | 6hr | 24hr | 48hr | 7d | Correlation with GeneChip data | SEQ ID NO. | Used restriction enzyme (MboI unless otherwise specified) |
| 101 | D17370_at | 742 | 559 | 182 | 861 | 844 | 1039 | 1 | 0.8 | *0.2* | 1.2 | 1.1 | 1.4 | 7 | | | | | | | | | |
| 102 | AJ011656cds_s_at | 262 | 389 | 53 | 406 | 354 | 266 | 1 | 1.5 | *0.2* | 1.5 | 1.4 | 1 | 7 | | | | | | | | | |
| 103 | rc_AI177004_s_at | 172 | 164 | 21 | 92 | 134 | 280 | 1 | 1 | *0.1* | 0.5 | 0.8 | 1.6 | 7 | 1.0 | 1.4 | *0.3* | 1.7 | 0.4 | 1.9 | 0.649 | 97 | |
| 104 | rc_AI172293_at | 284 | 286 | 85 | 192 | 182 | 491 | 1 | 1 | *0.3* | 0.7 | 0.6 | 1.7 | 7 | 1.0 | 1.3 | *0.2* | 1.2 | 0.5 | 1.3 | 0.788 | 98 | |
| 105 | rc_AA957498_at | 249 | 154 | 1 | 65 | 72 | 46 | 1 | 0.6 | *0* | *0.3* | *0.3* | *0.2* | 7 | | | | | | | | 99 | |
| 106 | rc_AI232087_at | 1152 | 1002 | 557 | 344 | 570 | 264 | 1 | 0.9 | 0.5 | *0.3* | 0.5 | *0.2* | 8 | | | | | | | | 100 | |
| 107 | M18363cds_s_at | 1447 | 1228 | 1113 | 358 | 599 | 342 | 1 | 0.8 | 0.8 | *0.2* | 0.4 | *0.2* | 8 | 1.0 | 1.4 | 1.0 | *0.1* | *0.2* | 1.6 | 0.326 | 101 | |
| 108 | J02585_at | 1386 | 899 | 1001 | 165 | 357 | 1453 | 1 | 0.6 | 0.7 | *0.1* | *0.3* | 1 | 8 | | | | | | | | 102 | *Nla* III |
| 109 | rc_AI044610_s_at | 482 | 447 | 294 | 100 | 389 | 357 | 1 | 0.9 | 0.6 | *0.2* | 0.8 | 0.7 | 8 | 1.0 | 1.3 | 0.9 | *0.1* | 1.4 | 1.3 | 0.828 | 103 | |
| 110 | X06150cds_g_at | 1605 | 1313 | 1180 | 511 | 972 | 1203 | 1 | 0.8 | 0.7 | *0.3* | 0.6 | 0.7 | 8 | 1.0 | 1.4 | 2.9 | 0.8 | 2.1 | 1.8 | 0.079 | 104 | |
| 111 | J05031_g_at | 470 | 428 | 366 | 115 | 179 | 233 | 1 | 0.9 | 0.8 | *0.2* | 0.4 | 0.5 | 8 | 1.0 | 1.6 | 1.1 | 0.6 | 1.0 | 1.5 | 0.499 | 105 | |
| 112 | rc_AA925393_at | 966 | 386 | 1465 | 162 | 311 | 860 | 1 | 0.4 | 1.5 | *0.2* | *0.3* | 0.9 | 8 | | | | | | | | 106 | |
| 113 | rc_AA926200_at | 1060 | 1588 | 602 | 120 | 684 | 983 | 1 | 1.5 | 0.6 | *0.1* | 0.6 | 0.9 | 8 | 1.1 | 1.4 | 0.9 | 0.4 | 1.8 | 2.9 | 0.370 | 107 | |
| 114 | AF037072_at | 909 | 787 | 627 | 91 | 148 | 140 | 1 | 0.9 | 0.7 | *0.1* | *0.2* | *0.2* | 8 | 1.0 | 1.1 | 0.6 | *0.0* | 0.4 | 0.8 | 0.822 | 108 | |
| 115 | rc_AA819899_at | 340 | 268 | 236 | 104 | 73 | 152 | 1 | 0.8 | 0.7 | *0.3* | *0.2* | 0.4 | 8 | 1.0 | 2.3 | 1.4 | 1.1 | 1.0 | 1.1 | 0.407 | 109 | |
| 116 | M22359mRNA_s_at | 481 | 724 | 375 | 149 | 99 | 247 | 1 | 1.5 | 0.8 | *0.3* | *0.2* | 0.5 | 8 | 1.0 | 1.6 | 1.1 | *0.3* | *0.2* | 1.8 | 0.629 | 110 | |
| 117 | K00996mRNA_s_at | 935 | 796 | 522 | 215 | 299 | 511 | 1 | 0.9 | 0.6 | *0.2* | *0.3* | 0.5 | 8 | 1.0 | 1.6 | 6.7 | 3.0 | 3.4 | 5.3 | -0.433 | 111 | |
| 118 | M59861_at | 611 | 565 | 311 | 178 | 283 | 425 | 1 | 0.9 | 0.5 | *0.3* | 0.5 | 0.7 | 8 | 1.0 | 1.7 | 1.0 | 0.3 | 1.1 | 1.9 | 0.582 | 112 | |
| 119 | rc_AI169656_at | 1437 | 1303 | 786 | 482 | 485 | 61 | 1 | 0.9 | 0.5 | *0.3* | *0.3* | *0* | 8 | | | | | | | | 113 | |
| 120 | X14552_at | 151 | 271 | 100 | 52 | 108 | 14 | 1 | 1.8 | 0.7 | *0.3* | 0.7 | *0.1* | 8 | | | | | | | | 114 | |

Table 1 (11)

| No. | GeneChip probe set | Gene expression intensity | | | | | | Ratio of expression level after hepatectomy to expression level before hepatectomy (0hr) (GeneChip) | | | | | | Group | Ratio of expression level after hepatectomy to expression level before hepatectomy (0hr) (ATAC) | | | | | | | Information about primers for ATAC-PCR | |
|---|---|---|---|---|---|---|---|---|---|---|---|---|---|---|---|---|---|---|---|---|---|---|---|
| | | 0hr | 1hr | 6hr | 24hr | 48hr | 7d | 0hr | 1hr | 6hr | 24hr | 48hr | 7d | | 0hr | 1hr | 6hr | 24hr | 48hr | 7d | Correlation with GeneChip data | SEQ ID NO. | Used restriction enzyme (MboI unless otherwise specified) |
| 121 | Y07534cds_s_at | 659 | 571 | 535 | 186 | 224 | 546 | 1 | 0.9 | 0.8 | *0.3* | *0.3* | 0.8 | 8 | 1.0 | 3.4 | 2.0 | 0.6 | 1.4 | 3.5 | 0.497 | 115 | |
| 122 | J02827_g_at | 234 | 212 | 171 | 20 | 144 | 132 | 1 | 0.9 | 0.7 | *0.1* | 0.6 | 0.6 | 8 | 1.0 | 1.1 | 1.2 | 0.5 | 1.0 | 2.3 | 0.262 | 116 | |
| 123 | K03249_at | 242 | 295 | 142 | 40 | 137 | 187 | 1 | 1.2 | 0.6 | *0.2* | 0.6 | 0.8 | 8 | | | | | | | | 117 | |
| 124 | rc_AI639418_at | 731 | 553 | 426 | 110 | 182 | 165 | 1 | 0.8 | 0.6 | *0.2* | *0.2* | *0.2* | 8 | 1.0 | 1.4 | 0.4 | 0.6 | *0.1* | 0.8 | 0.623 | 118 | |
| 125 | U88036_at | 674 | 819 | 553 | 123 | 642 | 568 | 1 | 1.2 | 0.8 | *0.2* | 1 | 0.8 | 8 | 1.0 | 1.1 | 0.8 | *0.1* | 0.4 | 0.7 | 0.805 | 119 | |
| 126 | X65296cds_s_at | 1046 | 1004 | 783 | 158 | 74 | 216 | 1 | 1 | 0.7 | *0.2* | *0.1* | *0.2* | 8 | | | | | | | | 120 | |
| 127 | D10354_s_at | 640 | 497 | 366 | 103 | 274 | 131 | 1 | 0.8 | 0.6 | *0.2* | 0.4 | *0.2* | 8 | 1 | 1.4 | 1.1 | 0.6 | 0.7 | 0.6 | 0.789 | 121 | |
| 128 | rc_AI144586_at | 459 | 344 | 213 | 132 | 148 | 176 | 1 | 0.7 | 0.5 | *0.3* | *0.3* | 0.4 | 8 | | | | | | | | 122 | |
| 129 | M84719_at | 295 | 728 | 182 | 37 | 80 | 63 | 1 | 2.5 | 0.6 | *0.1* | *0.3* | *0.2* | 8 | 1.0 | 2.7 | 0.7 | *0.3* | 0.4 | 1.0 | 0.954 | 123 | |
| 130 | K01934mRNA#2_at | 1125 | 982 | 837 | 124 | 212 | 1065 | 1 | 0.9 | 0.7 | *0.1* | *0.2* | 0.9 | 8 | | | | | | | | | |
| 131 | M77479_at | 1124 | 1103 | 738 | 355 | 661 | 705 | 1 | 1 | 0.7 | *0.3* | 0.6 | 0.6 | 8 | 1.0 | 1.9 | 1.0 | 0.6 | 1.1 | 2.0 | 0.442 | 124 | |
| 132 | J03863_at | 396 | 960 | 907 | 19 | 197 | 594 | 1 | 2.4 | 2.3 | *0* | 0.5 | 1.5 | 8 | 1 | 5.5 | 4.7 | 0.5 | 0.9 | 1.2 | 0.902 | 125 | |
| 133 | L22339_g_at | 1630 | 1305 | 1339 | 395 | 925 | 1263 | 1 | 0.8 | 0.8 | *0.2* | 0.6 | 0.8 | 8 | 1 | 1.2 | 0.8 | *0.1* | *0.3* | *0.2* | 0.709 | 126 | |
| 134 | X79081mRNA_f_at | 588 | 587 | 401 | 53 | 74 | 63 | 1 | 1 | 0.7 | *0.1* | *0.1* | *0.1* | 8 | | | | | | | | | |
| 135 | rc_AA945418_at | 3397 | 2633 | 2290 | 2194 | 699 | 1135 | 1 | 0.8 | 0.7 | 0.6 | *0.2* | *0.3* | 9 | | | | | | | | | |
| 136 | rc_AI231076_at | 675 | 789 | 891 | 510 | 144 | 529 | 1 | 1.2 | 1.3 | 0.8 | *0.2* | 0.8 | 9 | | | | | | | | | |
| 137 | U10697_s_at | 996 | 984 | 685 | 497 | 541 | 311 | 1 | 1 | 0.7 | 0.5 | 0.5 | *0.3* | 10 | | | | | | | | 127 | |

| | | |
|---|---|---|
| Correlation coefficient >= 0.7 | 34.000 | 45% |
| Correlation coefficient >= 0.5 | 50.000 | 67% |
| Number of genes measured by ATAC-PCR | 75.000 | |

<4> Gene expression analysis by ATAC-PCR

ATAC-PCR is a technique developed by Kato et al. (Kato, K et al., Nucl. Acids Res., 25, 4694-4696, 1997) and based on competitive RT-PCR using fluorescent primers. Quantitative expression analysis of a large number of genes can be conveniently carried out by ATAC-PCR. The experimental procedure of the ATAC-PCR method was according to the method of Kato et al. (Kato, K et al., Nucl. Acids Res., 25, 4694-4696, 1997).

First, double-stranded DNA was synthesized from cDNA synthesized by using a 5' biotinylated oligo dT primer and digested with a particular restriction enzyme (example utilizing MboI is explained herein). Subsequently, an adaptor having the same end sequence as that of the site digested with the restriction enzyme (6 types having different lengths were prepared) and the double-stranded DNA digested with the restriction enzyme MboI were ligated with DNA ligase. Among the 6 types of adaptors, 3 types were ligated to control cDNA (cDNA prepared from rat liver before hepatectomy) and mixed at a ratio of 10:3:1. The remaining 3 types of adaptors were ligated with cDNA prepared from the rat liver at 1, 6, 24, 48 hours and 7 days after the hepatectomy.

The ligation reaction products were mixed, 3' fragments of the double-stranded cDNA were recovered by using streptavidin-coated beads, and competitive RT-PCR was performed by using primers having a sequence common to all of the adaptors. The PCR products were analyzed by using ABI PRISM 3700 DNA Analyzer. This apparatus can separate fragments at each length by capillary electrophoresis and detect fluorescence intensity proportional to each expression level.

A calibration curve was prepared from fluorescence intensity of the PCR products obtained from the control, and gene expression change amounts in resected liver to be measured were calculated by using the calibration curve.

The nucleotide sequences of the used adaptors are shown below.

(Adaptor 1, SEQ ID NO: 128)

5'-GTACATATTGTCGTTAGAACGCG-3'

3'-CATGTATAACAGCAATCTTGCGCCTAG-5'

(Adaptor 2, SEQ ID NO: 129)

5'-GTACATATTGTCGTTAGAACGCGACT-3'

3'-CATGTATAACAGCAATCTTGCGCTGACTAG-5'

(Adaptor 3, SEQ ID NO: 130)

5'-GTACATATTGTCGTTAGAACGCGCATACT-3'

3'-CATGTATAACAGCAATCTTGCGCGTATGACTAG-5'

(Adaptor 4, SEQ ID NO: 131)

5'-GTACATATTGTCGTTAGAACGCGATCCATACT-3'

3'-CATGTATAACAGCAATCTTGCGCTAGGTATGACTAG 5'

(Adaptor 5, SEQ ID NO: 132)

5'-GTACATATTGTCGTTAGAACGCGTCAATCCATACT-3'

3'-CATGTATAACAGCAATCTTGCGCAGTTAGGTATGACTAG-5'

(Adaptor 6, SEQ ID NO: 133)

5'-GTACATATTGTCGTTAGAACGCGTACTCAATCCATACT-3'

3'-CATGTATAACAGCAATCTTGCGCATGAGTTAGGTATGACTAG-5'

As for the primer set used for the competitive PCR, the primer having the following nucleotide sequence was commonly used for the adaptor-side of all genes, and the sequences shown in Table 1 and Sequence Listing (shown in a direction from 5' to 3') were used as gene-specific primers.
(Adaptor-side primer)

5'-GTACATATTGTCGTTAGAACGC-3' (SEQ ID NO: 134)

For the genes of which expression changes were examined by ATAC-PCR using restriction enzymes other than MboI, names of the used restriction enzymes are shown in Table 1. Further, in such cases, adaptor sequences having an end sequence different from that used for MboI were used. Fig. 1 shows sequences of the different portions (boldfaces).

<5> Application of gene panel to screening for candidate liver regeneration accelerating substance

An experiment was performed to demonstrate that the gene panel of the present invention was effective for screening for liver regeneration accelerating substances.

L-alanine is known to have effect of accelerating liver regeneration (Maezono K et al., Hepatology 24(5), 1996, Effect of Alanine on D-Galactosamine-Induced Acute Liver Failure in Rats; Japanese Patent Laid-open Publication No. 5-229940). Therefore, rats were subjected to partial hepatectomy and orally administered with L-alanine at 18 and 21 hours after the hepatectomy, and then gene expressions at 24 hours after the hepatectomy were examined.

Five 6 week-old F344 male rats (120 g) were preliminarily fed for 6 days by giving feed CRF-1 (Oriental Yeast) and water and then divided into 2 groups (Group 1 and Group 2) each consisting of rats with the same body weight. At 24 hours before dissection, 70% partial hepatectomy was performed by the method by Higgins-Anderson (Higgins, GM and Anderson, RM, Arch. Pathol. 12, 186-191, 1931). On the day of autopsy, the rats were starved for 6 hours before dissection. At 6 and 3 hours before dissection, 2 g/10 ml/kg of L-alanine was orally administered as an aqueous solution containing 0.3% aqueous carboxymethylcellulose (CMC).

After the rats were killed by bleeding under anesthesia, the dissection was performed. Liver was extracted, weighed, cryopreserved, and then subjected to the mRNA expression measurement by the ATAC-PCR method. Further, liver samples were prepared to measure the liver proliferating cell nucleus antigen (PCNA) levels.

Gene expressions in rat liver in each group were measured by ATAC-PCR. The preparation of RNA and ATAC-PCR were performed as described above. In ATAC-PCR, among the genes in the gene panel, 117 types of which expression could be measured by ATAC-PCR using the restriction enzyme MboI were measured (those except for Nos. 12, 49, 52, 55, 58, 59, 61, 69, 73, 77, 83, 90, 93, 101, 102, 108, 130, 134, 135 and 136 mentioned in Table 1).

Further, the livers of rats administered with L-alanine were stained by using anti-PCNA antibodies (antibodies directed to proliferating cell nuclear antigen (PCNA), a replication factor that accelerates activity of DNA polymerase a), and the PCNA labeling index was measured by the method of Tanaka et al. (Tanaka et al., Byori to Rinsho, 9 (6): 791-798, 1991).

The measurement results of the PCNA labeling index are shown in Table 2. Due to the L-alanine administration, the PCNA labeling index in the livers elevated as compared with the livers of the non-administered rats (Table 2). In Table 2, Ala+ represents the L-alanine-administered group, and Ala-represents the L-alanine non-administered group. Thus, hepatocytes proliferation accelerating effect due to the L-alanine administration was observed.

Table 2

| | PCNA positive cells | PCNA Negative cells | Labeling Index (%) |
|---|---|---|---|
| 1-1<br>1-2<br>1-3 | 4<br>5<br>2 | 570<br>578<br>658 | 0.7%<br>0.9%<br>0.3% |
| Mean<br>SD | | | 0.6%<br>0.3% |
| 2-1<br>2-2 | 468<br>426 | 247<br>240 | 65.5%<br>64.0% |
| Mean<br>SD | | | 64.7%<br>1.1% |
| 1-1 to 1-3: Group 1<br>2-1, 2-2: Group 1 | | | |

Further, the gene expressions in Groups 1 and 2 were measured by ATAC-PCR for the genes of the gene panel and compared. The expression levels were obtained as relative values of Group 1 based on the values of Group 1 (Ala+/Ala- in Table 3). As a result, there were found 7 types of genes which showed 3 times or more of expression diffence between the L-alanine-administered group and L-alanine non-administered group (Table 3).

Table 3

| Number | Ala+, 24hr/Ala-, 24hr |
|---|---|
| 5 | 4.8 |
| 17 | 5.5 |
| 36 | 4.6 |
| 46 | 4.4 |
| 67 | 4.2 |
| 68 | 3.5 |
| 117 | 3.2 |

Subsequently, to compare expression changes of the genes in the gene panel and gene expression changes due to the L-alanine administration, the ratios of expressions of the genes in Group 2 to expressions of the genes in the gene panel at 0 hour after the hepatectomy were calculated. The results are shown in Table 4. In Table 4, 1hr, 6hr, 24hr, 48hr and 7d represent relative expression values in the remaining livers based on the gene expression in the liver before the hepatectomy at 1, 6, 24, 48 hours and 7 days after the hepatectomy, respectively. When the expression values of Ala- at 24hr and Ala+ at 24hr are compared, marked differences (3 times or more) in the expression levels were observed in both of Groups 1 and 2 for 7 types (Nos. 5, 17, 36, 46, 67, 68 and 117) among the genes in the gene panel (Table 4). Among these, the results of 5 types (Nos. 17, 36, 46, 67 and 68) corelated with the results of the genes in the gene panel. That is, for the genes in the gene panel which showed expression increases from the levels before the hepatectomy at 24 hours, expressions exceeding the increased expressions were observed due to the L-alanine administration. On the contrary, for the genes in the gene panel which showed expression decreases from the levels before hepatectomy at 24 hours, the expressions less than the decreased expressions were observed due to the L-alanine administration.

For the remaining 2 types ( Nos. 5 and 117) other than the above, the genes of the gene panel did not show increased or decreased expression change due to the L-alanine administration at 24 hours. However, for No. 5, expression increase was observed due to the L-alanine administration at the maximum expression times (6 hr and 1 hr). As a result, it can be construed that expressions higher than the expressions of the genes in the gene panel were maintained at 24 hours. On the other hand, since No. 117 showed decreased change (decrease of expression) with the L-alanine administration compared with that observed without L-alanine administration at 24 hours, it is determined that it is not suitable for use in screening for candidate liver regeneration accelerating substance.

Table 4

| Number | Ala- | | | | | Ala+ |
|---|---|---|---|---|---|---|
| | 1hr | 6hr | 24hr | 48hr | 7d | 24hr |
| 5 | 10.3 | 14.1 | 1 | 1.4 | 2.3 | 4.8 |
| 17 | 10 | 8.3 | 41.5 | 61.3 | 28 | 228.3 |
| 36 | 1 | 3.2 | 5.6 | 5.4 | 3.3 | 25.8 |
| 46 | 1.3 | 5.4 | 2.6 | 4.3 | 3.2 | 11.4 |
| 67 | 1.5 | 0.4 | 6.1 | 10.4 | 4.8 | 25.6 |
| 68 | 1.3 | 1 | 7.1 | 8 | 3.3 | 24.9 |
| 117 | 0.9 | 0.6 | 0.2 | 0.3 | 0.5 | 0.6 |

Based on the above results, it can be considered that, in screening utilizing the gene panel of the present invention, if a substance provides marked expression changes (3 times or more) of about 6 genes and the changes corelate to those in the gene panel (genes showing expression increase in the gene panel show further expression increases, and genes showing expression decrease in the gene panel show further expression decreases), such a substance can be selected as a candidate liver regeneration accelerating substance.

Example 2: Expression analysis of genes involved in L-alanine metabolism during liver regeneration by Tagman PCR (SYBR Green).

In the same manner as in the above <1> and <2>, total RNA was purified from the remaining livers (regenerating livers) at 0, 1, 6, 24, 48 and 168 hours after the partial hepatectomy of rats, and changes in expression levels of various genes were examined by using the Taqman PCR (SYBR Green) method.

(1) Preparation of template

The synthesis of template cDNA used for Taqman PCR (SYBR Green) was performed by using Superscript First-Strand Synthesis System for RT-PCR (GIBCO BRL). In an amount of 500 ng of total RNA, 1 µl of 0.5 µg/µl Oligo $(dT)_{12-18}$ and 1 µl of 10 mM dNTP mix were dissolved in DEPC-treated water to obtain a total volume of 10 µl. A reaction was allowed at 65°C for 5 minutes, and the reaction mixture was cooled on ice, added and mixed with 2 µl of 10 x RT buffer, 4 µl of 25 mM $MgCl_2$ , 2 µl of 0.1 M DTT and 1 µl of RNase Inhibitor, and kept warm at 42°C for 2 minutes. The reaction mixture was added with 1 µl (50 units) of reverse transcriptase (Superscript II RT), and a reaction was allowed at 42°C for 50 minutes and further at 70°C for 15 minutes.

(2) Selection of candidate gene and design of primer

The expression profiles of the genes involved in L-alanine metabolism during liver regeneration were examined. To exhaustively extract rat genes involved in L-alanine metabolism, the external database UniGene (http://www.ncbi.nlm.nih.gov/UniGene) was searched with keywords related to amino acid transporters, TCA cycle, respiratory chain and various carriers, and 59 types of genes of which sequences were known or for which primers could be designed were retrieved. Among these, 42 types were among the genes analyzed by using GeneChip in Example 1.

The primers used in Taqman PCR (SYBR Green) were designed by using the external database Primer3 (http://www-genome.wi.mit.edu/cgi-bin/primer/primer3_www.cgi). The names and Unigene Nos. of the genes and the nucleotide sequences of their primers are shown in Table 6. The table includes only the genes of which expression changes were observed as a result of the analysis by Taqman PCR (SYBR Green).

(3) Taqman PCR (SYBR Green)

Taqman PCR (SYBR Green) was performed by the SYBR Green method (Schmittgen TD et al., Analytical Biochem., 285, 194-204, 2000). Specifically, the reaction was performed as follows. A reaction mixture having the composition shown in Table 5 was mixed in a PCR tube for Taqman, and PCR was performed by using ABI 7700 Prism Sequence Detector (ABI). The rection was performed with a cycle consisting of reactions at 50°C for 2 minutes and at 95°C for 10 minutes followed by reactions at 95°C for 15 seconds and at 60°C for 1 minute, which was repeated 40 times.

Table 5

| Composition of PCR reaction mixture (per tube) | |
|---|---|
| Template cDNA (corresponding to 2.5 ng of total RNA) | 0.1 µl |
| 5 units/µl AmpliTaq Gold | 0.05 µl |
| dNTP mix (2.5 mM each) | 0.8 µl |
| 25 mM $MgCl_2$ | 1.2 µl |
| 10 x SYBR buffer | 1 µl |
| Primer solution (forward) | 1 µl |
| Primer solution (reverse) | 1 µl |
| Distilled water | |
| | Total: 10 µl |

(3) Data Analysis

All the Taqman PCR (SYBR Green) reactions were performed in duplicate, and the cycle threshold (CT) was calculated. The expression level at CT = 30 was defined as 1, and a relative expression level was obtained according to the following equation. "CT sample" represents CT of a gene measured.

$$\text{Relative expression level} = 2^{(30\ -\ \text{CT sample})}$$

Further, a relative value of the expression level of each gene was obtained based on the relative expression level of β-actin, which was taken as 1000. The results of the genes of which expressions changed in regenerating livers compared with those in normal liver are shown in Table 6. It is noted that, among the genes which showed expression change in regenerating livers in Example 1, expression changes were not reproduced for the genes of Nos. 81 (Rn. 29771) and 131 (Rn.9913) mentioned in Table 1.

Table 6

| No. | Unigene No. (Rn.) | Gene | Primer sequence (SEQ ID NO.) | | Ratio of expression level after hepatectomy to expression level before hepatectomy (0hr) | | | | | |
|---|---|---|---|---|---|---|---|---|---|---|
| | | | 5' end | 3' end | 0hr | 1hr | 6hr | 24hr | 48hr | 7d |
| 138 | 32067 | Transporter protein; system N1 Na+ and H+-coupled glutamine transporter | 135 | 136 | 1.00 | 0.96 | 0.60 | 0.60 | 0.37 | 0.86 |
| 139 | 53981 | Acetyl-CoA transporter | 137 | 138 | 1.00 | 0.95 | 0.39 | 0.04 | 0.29 | 0.82 |
| 140 | 54434 | Organic cation transporter OCTN1 | 139 | 140 | 1.00 | 0.98 | 0.04 | 0.07 | 0.13 | 0.58 |
| 141 | 14539 | RAT BILE SALT EXPORT PUMP (ATP-BINDING CASSETTE, SUB-FAMILY B | 141 | 142 | 1.00 | 0.95 | 0.55 | 0.34 | 0.58 | 0.79 |
| 142 | 29977 | MRP3 RAT CANALICULAR MULTISPECIFIC ORGANIC ANION TRANSPORTER 2 | 143 | 144 | 1.00 | 0.70 | 1.25 | 1.30 | 3.78 | 0.70 |
| 143 | 10240 | Solute carrier family 1, member 2 | 145 | 146 | 1.00 | 2.74 | 0.45 | 0.17 | 0.60 | 1.51 |
| 144 | 23204 | Rattus norvegicus mRNA for LRp105, complete cds | 147 | 148 | 1.00 | 1.62 | 0.88 | 0.24 | 0.32 | 1.01 |
| 145 | 11094 | Pyruvate carboxylase | 149 | 150 | 1.00 | 1.14 | 0.40 | 0.11 | 0.36 | 0.78 |
| 146 | 11363 | Pyruvate dehydrogenase kinase 2 subunit p45 (PDK2) | 151 | 152 | 1.00 | 1.07 | 0.50 | 0.20 | 0.31 | 0.98 |
| 147 | 3766 | Rat mitochondrial succinyl-CoA synthetase alpha subunit (cytoplasmic precursor) mRNA, complete cds | 153 | 154 | 1.00 | 1.03 | 0.74 | 0.57 | 0.98 | 0.78 |
| 148 | 3628 | Glutamate oxaloacetate transaminase 2, mitochondrial (aspartate aminotransferase 2) | 155 | 156 | 1.00 | 0.75 | 0.63 | 0.48 | 0.68 | 0.83 |
| 149 | 6085 | Solute carrier 16 (monocarboxylic acid transporter), member 1 | 157 | 158 | 1.00 | 0.46 | 1.50 | 0.84 | 0.93 | 0.72 |
| 150 | 8368 | Solute carrier family 25 (mitochondrial carrier; citrate transporter) member 1 | 159 | 160 | 1.00 | 0.55 | 0.40 | 0.57 | 0.60 | 0.85 |
| 151 | 5819 | Glutamic-oxaloacetic transaminase 1, soluble (aspartate aminotransferase, cytosolic) see also D1Mgh12 | 161 | 162 | 1.00 | 3.18 | 11.32 | 1.40 | 0.69 | 1.33 |

Example 3: Expression analysis of genes involved in L-alanine metabolism by Taqman PCR (SYBR Green) with L-alanine administration during liver regeneration

Expression changes were examined with L-alanine administration during liver regeneration regarding 59 types of rat genes involved in L-alanine metabolism extracted in Example 2.

(1) Preparation of regenerating liver

Five 6 week-old F344 male rats (120 g) were fed for 6 days by giving feed CRF-1 (Oriental Yeast) and water and then divided into 2 groups (Group 1 and Group 2) each consisting of rats with the same body weight. At 24 hours before dissection, 70% partial hepatectomy (left lobe and intermediate lobe) was performed by the method of Higgins-Anderson (Higgins, GM and Anderson, RM, Arch. Pathol. 12, 186-191, 1931). On the day of autopsy, the rats were starved for 6 hours before the dissection. At 18 and 21 hours after the partial hepatectomy, 2 g/10 ml/kg BW of L-alanine was orally administered as an aqueous solution containing 0.3% carboxymethylcellulose (Group 1), and only 0.3% carboxymethylcellulose was orally administered as a control (Group 2).

Dissection was performed at 24 hours after the partial hepatectomy. After the rats were killed by bleeding under anesthesia, livers were extracted, weighed, cryopreserved and then subjected to the mRNA expression measurement by the Taqman PCR (SYBR Green) method.

(2) Purification of total RNA

Total RNA was purified from the remaining liver of 70% partially hepatectomized rats in the L-alanine administered group (Group 1) and the control group (Group 2) in the same manner as in Example 1.

(3) Synthesis of template

Templante cDNA used in Taqman PCR (SYBR Green) was synthesized in the same manner as in Example 2.

(4) Desigan of primers

Primers used in Taqman PCR (SYBR Green) were designed by using the external database Primer3 (http://www-genome.wi.mit.edu/cgi-bin/primer/primer3_www.cgi). The names and Unigene Nos. of the genes and the nucleotide sequences of their primers are shown in Table 7. The table includes only the genes for which expression changes were observed as a result of the analysis by Taqman PCR (SYBR Green).

(5) Taqman PCR (SYBR Green)

Taqman PCR (SYBR Green) was performed in the same manner as in Example 2.

(6) Data analysis

All the Taqman PCR (SYBR Green) reactions were performed in duplicate, and the cycle threshold (CT) was calculated. The expression level at CT = 30 was defined as 1, and a relative expression level was obtained according to the following equation.

$$\text{Relative expression level} = 2^{(30\ -\ \text{CT sample})}$$

Futher, a relative value of the expression level of each gene was obtained based on the relative expression of β-Actin, which was taken as 1000. The results of genes for which changes in expression level due to the L-alanine administration were observed are shown in Figs. 2 to 4. Among these genes, expression changes were also observed in 4 types of genes (Nos. 139, 140 and 151 mentioned in Table 6 and No. 166 mentioned in Table 7) among the genes of which expressions changed in regenerating liver in Example 2.

Industrial Applicability

The present invention provides expression information of genes involved in liver regeneration. By utilizing this expression information, drugs which accelerate liver regeneration or the like can be screened.

Table 7

| No. | UrigeneNO. (Rh.) | Gene | Primer sequence (SEQ ID NO) | |
|---|---|---|---|---|
| | | | 5' end | 3' end |
| 152 | 48707 | Cationic amino acid transporter-2A | 163 | 164 |
| 153 | 29782 | Fumarate hydratase | 165 | 166 |
| 154 | 1093 | Rattus norvegicus mRNA for NAD+specific isocitrate dehydrogenase b-subunit, partial cds | 167 | 168 |
| 155 | 2837 | NAD (H)-spedfic isocitrate dehydrogenase gamma subunit | 169 | 170 |
| 156 | 1504 | Rattus norvegicuscytosolic malate dehydrogenase (Ndh) mRNA compl ete cds | 171 | 172 |
| 157 | 88C | Cytochrome c oxidase subunit Via (liver) | 173 | 174 |
| 158 | 1745 | Cytochrome c oxidase subunit VIIa 3 | 175 | 176 |
| 159 | 2270 | Rattus norvegicus liver cytochrome c oxidase subunit VIII (COX-VIII) mRNA 3' end of cds | 177 | 178 |
| 160 | 19207 | Rattus norvegicus MRNA for cytochrome C oxidase assembly protein COX17, complete cds | 179 | 180 |
| 161 | 10249 | Cytochrome b5, outer mitochondrial membrane isoform | 181 | 182 |
| 162 | 8C | ATP synthase subunit d | 183 | 184 |
| 163 | 3357 | ATP swtjmse, H+ transporting mitochondrial F0 complex, subunit c (subunit 9), isoform 1 | 185 | 186 |
| 164 | 9723 | Rattus norvegicus (clone gamma-3) ATP synthase gamma-subunit (ATP5c) mRNA 3' end cds | 187 | 188 |
| 165 | 853 | 2-oxoglutarate carrier | 189 | 190 |
| 166 | 3211 0 | Rattus norvegicus glycine transporter mRNA, complete cds | 191 | 192 |

SEQUENCE LISTING

<110> Ajinomoto Co., Inc.

<120> Gene Panel for Genes Involving Liver Regeneration

<130> B868AY0P1331

<140>
<141> 2002-03-13

<150> JP 2001-070940
<151> 2001-03-13

<160> 192

<170> PatentIn Ver. 2.0

<210> 1
<211> 21
<212> DNA
<213> Artificial Sequence

<220>
<223> Description of Artificial Sequence: primer

<400> 1
cagaatctgt caatcacctt c 21

<210> 2
<211> 20
<212> DNA
<213> Artificial Sequence

<220>
<223> Description of Artificial Sequence: primer

<400> 2
caaccatcac cgcacaagaa 20

<210> 3

<211> 19
<212> DNA
<213> Artificial Sequence

<220>
<223> Description of Artificial Sequence: primer

<400> 3
ggccatattt cgagcatcc 20

<210> 4
<211>
<212> DNA
<213> Artificial Sequence

<220>
<223> Description of Artificial Sequence: primer

<400> 4
tcctgagcgacttccac

<210> 5
<211> 16
<212> DNA
<213> Artificial Sequence

<220>
<223> Description of Artificial Sequence: primer

<400> 5
tggaatttgg cctgaa 16

<210> 6
<211>
<212> DNA
<213> Artificial Sequence

<220>
<223> Description of Artificial Sequence: primer

<400> 6
ccatttgttcgccaac

<210> 7
<211> 21
<212> DNA
<213> Artificial Sequence

<220>
<223> Description of Artificial Sequence: primer

<400> 7
atgtagcact gaggtattct g 21

<210> 8
<211> 20
<212> DNA
<213> Artificial Sequence

<220>
<223> Description of Artificial Sequence: primer

<400> 8
ccaggattag cttcataacc 20

<210> 9
<211> 22
<212> DNA
<213> Artificial Sequence

<220>
<223> Description of Artificial Sequence: primer

<400> 9
actctgctta tccactgact at 22

<210> 10
<211> 17
<212> DNA
<213> Artificial Sequence

<220>
<223> Description of Artificial Sequence: primer

<400> 10
gctggttcgt atcctgg 17

<210> 11
<211> 20
<212> DNA
<213> Artificial Sequence

<220>
<223> Description of Artificial Sequence: primer
<400> 11
taggacaggc acaaagttcg 20

<210> 12
<211> 18
<212> DNA
<213> Artificial Sequence

<220>
<223> Description of Artificial Sequence: primer

<400> 12
atgggtaggc tgggcaag 19

<210> 13
<211> 20
<212> DNA
<213> Artificial Sequence

<220>
<223> Description of Artificial Sequence: primer

<400> 13
ggatttcttg gtcttgctgc 20

<210> 14
<211> 18
<212> DNA
<213> Artificial Sequence

<220>
<223> Description of Artificial Sequence: primer

<400> 14
aagactaccg cagaggtg 18

<210> 15
<211> 18
<212> DNA
<213> Artificial Sequence

<220>
<223> Description of Artificial Sequence: primer

<400> 15
acttcgcaca gcccacgg 18

<210> 16
<211> 21
<212> DNA
<213> Artificial Sequence

<220>
<223> Description of Artificial Sequence: primer

<400> 16
agagtgtttg gtaagtagca c 21

<210> 17
<211> 21
<212> DNA
<213> Artificial Sequence

<220>
<223> Description of Artificial Sequence: primer

<400> 17
ccagtttctt tgcagtatct c 21

<210> 18
<211> 22
<212> DNA
<213> Artificial Sequence

<220>
<223> Description of Artificial Sequence: primer

<400> 18

aatgtcataa tttattctgc tg 22

<210> 19
<211> 19
<212> DNA
<213> Artificial Sequence

<220>
<223> Description of Artificial Sequence: primer

<400> 19
cactgaggaa ggaggaagg 19

<210> 20
<211> 20
<212> DNA
<213> Artificial Sequence

<220>
<223> Description of Artificial Sequence: primer

<400> 20
aacaactttc agttttgcac 20

<210> 21
<211> 18
<212> DNA
<213> Artificial Sequence

<220>
<223> Description of Artificial Sequence: primer

<400> 21
gcatcgagca tcacaggg 18

<210> 22
<211> 17
<212> DNA
<213> Artificial Sequence

<220>
<223> Description of Artificial Sequence: primer

<400> 22
gtaaggaccc agctgct 17

<210> 23
<211> 20
<212> DNA
<213> Artificial Sequence

<220>
<223> Description of Artificial Sequence: primer

<400> 23
gcagatgaca gccagacaat 20

<210> 24
<211> 19
<212> DNA
<213> Artificial Sequence

<220>
<223> Description of Artificial Sequence: primer

<400> 24
aaatgaaaca atcccatca 19

<210> 25
<211> 19
<212> DNA
<213> Artificial Sequence

<220>
<223> Description of Artificial Sequence: primer

<400> 25
gtaaaagtac tgtcccgga 19

<210> 26
<211> 20
<212> DNA
<213> Artificial Sequence

<220>
<223> Description of Artificial Sequence: primer

<400> 26
gtagttcatc atctgcggtg 20

<210> 27
<211> 18
<212> DNA
<213> Artificial Sequence

<220>
<223> Description of Artificial Sequence: primer

<400> 27
cgtgtgttgc gtcagtcc 18

<210> 28
<211> 19
<212> DNA
<213> Artificial Sequence

<220>
<223> Description of Artificial Sequence: primer

<400> 28
acactctgac acatgctcc 19

<210> 29
<211> 20
<212> DNA
<213> Artificial Sequence

<220>
<223> Description of Artificial Sequence: primer

<400> 29
cacatcacca ttttatcgat 20

<210> 30
<211> 15
<212> DNA
<213> Artificial Sequence

<220>

<223> Description of Artificial Sequence: primer

<400> 30
tcttgtcccc tccca 15

<210> 31
<211> 23
<212> DNA
<213> Artificial Sequence

<220>
<223> Description of Artificial Sequence: primer

<400> 31
acagattgat tgaaccattg cag 23

<210> 32
<211> 20
<212> DNA
<213> Artificial Sequence

<220>
<223> Description of Artificial Sequence: primer

<400> 32
atggcgttga agatgtgcag 20

<210> 33
<211> 16
<212> DNA
<213> Artificial Sequence

<220>
<223> Description of Artificial Sequence: primer

<400> 33
ttgcccaagt gtccag 16

<210> 34
<211> 19
<212> DNA
<213> Artificial Sequence

<220>
<223> Description of Artificial Sequence: primer

<400> 34
aaatcttagg ggtgaaaac 19

<210> 35
<211> 22
<212> DNA
<213> Artificial Sequence

<220>
<223> Description of Artificial Sequence: primer

<400> 35
tagtggtaac atttaaccca tg 22

<210> 36
<211> 22
<212> DNA
<213> Artificial Sequence

<220>
<223> Description of Artificial Sequence: primer

<400> 36
tggaacaata aaaagagagg cc 22

<210> 37
<211> 20
<212> DNA
<213> Artificial Sequence

<220>
<223> Description of Artificial Sequence: primer

<400> 37
tcaatagcaa gtgggtaggc 20

<210> 38
<211> 20
<212> DNA
<213> Artificial Sequence

<220>
<223> Description of Artificial Sequence: primer

<400> 38
cacccccata agaactattg 20

<210> 39
<211> 22
<212> DNA
<213> Artificial Sequence

<220>
<223> Description of Artificial Sequence: primer

<400> 39
cattattcaa ctattcctca gc 22

<210> 40
<211> 19
<212> DNA
<213> Artificial Sequence

<220>
<223> Description of Artificial Sequence: primer

<400> 40
ctggctgtga ctatgggac 19

<210> 41
<211> 21
<212> DNA
<213> Artificial Sequence

<220>
<223> Description of Artificial Sequence: primer

<400> 41
aacctttcct acgccttttg g 21

<210> 42
<211> 20
<212> DNA

<213> Artificial Sequence

<220>
<223> Description of Artificial Sequence: primer

<400> 42
ttcacgttac cttcctgtgc 20

<210> 43
<211> 20
<212> DNA
<213> Artificial Sequence

<220>
<223> Description of Artificial Sequence: primer

<400> 43
ataaattccc actgccacgg 20

<210> 44
<211> 20
<212> DNA
<213> Artificial Sequence

<220>
<223> Description of Artificial Sequence: primer

<400> 44
tcctctcctt tctccatcag 20

<210> 45
<211> 17
<212> DNA
<213> Artificial Sequence

<220>
<223> Description of Artificial Sequence: primer

<400> 45
ggttggctcc tcatagg 17

<210> 46
<211> 21

<212> DNA
<213> Artificial Sequence

<220>
<223> Description of Artificial Sequence: primer

<400> 46
cttctacaca gactgccaca g 21

<210> 47
<211> 17
<212> DNA
<213> Artificial Sequence

<220>
<223> Description of Artificial Sequence: primer

<400> 47
tgtgcacttc ctgcaca 17

<210> 48
<211> 18
<212> DNA
<213> Artificial Sequence

<220>
<223> Description of Artificial Sequence: primer

<400> 48
gagagcgcgt acacagac 18

<210> 49
<211> 18
<212> DNA
<213> Artificial Sequence

<220>
<223> Description of Artificial Sequence: primer

<400> 49
aacggcttgg aggatggc 18

<210> 50

<211> 20
<212> DNA
<213> Artificial Sequence

<220>
<223> Description of Artificial Sequence: primer

<400> 50
taagaagtgg catactgctc 20

<210> 51
<211> 18
<212> DNA
<213> Artificial Sequence

<220>
<223> Description of Artificial Sequence: primer

<400> 51
caggtgacag gattacgg 18

<210> 52
<211> 16
<212> DNA
<213> Artificial Sequence

<220>
<223> Description of Artificial Sequence: primer

<400> 52
ggcaggttca aacgct 16

<210> 53
<211> 20
<212> DNA
<213> Artificial Sequence

<220>
<223> Description of Artificial Sequence: primer

<400> 53
tggaaaagaa ctgaaggcgc 20

<210> 54
<211> 20
<212> DNA
<213> Artificial Sequence

<220>
<223> Description of Artificial Sequence: primer

<400> 54
catcttacca tttctactgcac 20

<210> 55
<211> 20
<212> DNA
<213> Artificial Sequence

<220>
<223> Description of Artificial Sequence: primer

<400> 55
tcataaagac acagcaccag 20

<210> 56
<211> 20
<212> DNA
<213> Artificial Sequence

<220>
<223> Description of Artificial Sequence: primer

<400> 56
tatggagaga gacgaggttg 20

<210> 57
<211> 20
<212> DNA
<213> Artificial Sequence

<220>
<223> Description of Artificial Sequence: primer

<400> 57
tgaaggtgac tgccatcttg 20

<210> 58
<211> 20
<212> DNA
<213> Artificial Sequence

<220>
<223> Description of Artificial Sequence: primer

<400> 58
agagagacac atggacagac 20

<210> 59
<211> 20
<212> DNA
<213> Artificial Sequence

<220>
<223> Description of Artificial Sequence: primer

<400> 59
agacaggaat gaagcacagc 20

<210> 60
<211> 19
<212> DNA
<213> Artificial Sequence

<220>
<223> Description of Artificial Sequence: primer

<400> 60
tgtatgacaa atgcagcag 19

<210> 61
<211> 20
<212> DNA
<213> Artificial Sequence

<220>
<223> Description of Artificial Sequence: primer

<400> 61

aactgtggct atgacagtcc 20

<210> 62
<211> 18
<212> DNA
<213> Artificial Sequence

<220>
<223> Description of Artificial Sequence: primer

<400> 62
actgttccga ctctgggc 18

<210> 63
<211> 18
<212> DNA
<213> Artificial Sequence

<220>
<223> Description of Artificial Sequence: primer

<400> 63
ctcgcataga cagccagg 18

<210> 64
<211> 21
<212> DNA
<213> Artificial Sequence

<220>
<223> Description of Artificial Sequence: primer

<400> 64
tattaaccat tccagtacag t 21

<210> 65
<211> 19
<212> DNA
<213> Artificial Sequence

<220>
<223> Description of Artificial Sequence: primer

<400> 65
tttgcacctc ctctaagtc 19

<210> 66
<211> 20
<212> DNA
<213> Artificial Sequence

<220>
<223> Description of Artificial Sequence: primer

<400> 66
atcttgctga accgtgaagg 20

<210> 67
<211> 18
<212> DNA
<213> Artificial Sequence

<220>
<223> Description of Artificial Sequence: primer

<400> 67
gggtgtgcaa gccagacc 18

<210> 68
<211> 19
<212> DNA
<213> Artificial Sequence

<220>
<223> Description of Artificial Sequence: primer

<400> 68
ccatggagaa atagatgcc 19

<210> 69
<211> 18
<212> DNA
<213> Artificial Sequence

<220>
<223> Description of Artificial Sequence: primer

<400> 69
gtgaagtgga cggctcgg 18

<210> 70
<211> 18
<212> DNA
<213> Artificial Sequence

<220>
<223> Description of Artificial Sequence: primer

<400> 70
ctctgcggtg aggtactc 18

<210> 71
<211> 21
<212> DNA
<213> Artificial Sequence

<220>
<223> Description of Artificial Sequence: primer

<400> 71
cagaaattga agtccactca g 21

<210> 72
<211> 20
<212> DNA
<213> Artificial Sequence

<220>
<223> Description of Artificial Sequence: primer

<400> 72
aatttgtcca gagaggcgtg 20

<210> 73
<211> 20
<212> DNA
<213> Artificial Sequence

<220>

<223> Description of Artificial Sequence: primer

<400> 73
ggaagtcaaa gacctgaatg 20

<210> 74
<211> 23
<212> DNA
<213> Artificial Sequence

<220>
<223> Description of Artificial Sequence: primer

<400> 74
caacataaac ataaacatca gtg 23

<210> 75
<211> 20
<212> DNA
<213> Artificial Sequence

<220>
<223> Description of Artificial Sequence: primer

<400> 75
atttctccct tccctcaacc 20

<210> 76
<211> 18
<212> DNA
<213> Artificial Sequence

<220>
<223> Description of Artificial Sequence: primer

<400> 76
gtccatccca gccaccag 18

<210> 77
<211> 18
<212> DNA
<213> Artificial Sequence

<220>
<223> Description of Artificial Sequence: primer

<400> 77
tggagcagga gcagttgg 18

<210> 78
<211> 20
<212> DNA
<213> Artificial Sequence

<220>
<223> Description of Artificial Sequence: primer

<400> 78
ttggtccgaa acacagaacg 20

<210> 79
<211> 19
<212> DNA
<213> Artificial Sequence

<220>
<223> Description of Artificial Sequence: primer

<400> 79
actgtggacc tgagttctg 19

<210> 80
<211> 16
<212> DNA
<213> Artificial Sequence

<220>
<223> Description of Artificial Sequence: primer

<400> 80
ggcaggttca aacgct 16

<210> 81
<211> 20
<212> DNA
<213> Artificial Sequence

<220>
<223> Description of Artificial Sequence: primer

<400> 81
agtctcaagg ttttattggc 20

<210> 82
<211> 19
<212> DNA
<213> Artificial Sequence

<220>
<223> Description of Artificial Sequence: primer

<400> 82
cagctatgta agcaccgaa 19

<210> 83
<211> 17
<212> DNA
<213> Artificial Sequence

<220>
<223> Description of Artificial Sequence: primer

<400> 83
tgctggtacc gtgagct 17

<210> 84
<211> 18
<212> DNA
<213> Artificial Sequence

<220>
<223> Description of Artificial Sequence: primer

<400> 84
atttcccacc ctacccgg 18

<210> 85
<211> 20
<212> DNA

<213> Artificial Sequence

<220>
<223> Description of Artificial Sequence: primer

<400> 85
atgagttctt ggctggcttg 20

<210> 86
<211> 22
<212> DNA
<213> Artificial Sequence

<220>
<223> Description of Artificial Sequence: primer

<400> 86
gtggttacaa attcttcact gc 22

<210> 87
<211> 20
<212> DNA
<213> Artificial Sequence

<220>
<223> Description of Artificial Sequence: primer

<400> 87
cttagatgct gagagagtgg 20

<210> 88
<211> 20
<212> DNA
<213> Artificial Sequence

<220>
<223> Description of Artificial Sequence: primer

<400> 88
gtgacttagc tttacccttc 20

<210> 89
<211> 17

<212> DNA
<213> Artificial Sequence

<220>
<223> Description of Artificial Sequence: primer

<400> 89
ccgtttagct tggtgga 17

<210> 90
<211> 16
<212> DNA
<213> Artificial Sequence

<220>
<223> Description of Artificial Sequence: primer

<400> 90
tttcctgcac accctc 16

<210> 91
<211> 20
<212> DNA
<213> Artificial Sequence

<220>
<223> Description of Artificial Sequence: primer

<400> 91
agcaattcca acaccaaggc 20

<210> 92
<211> 20
<212> DNA
<213> Artificial Sequence

<220>
<223> Description of Artificial Sequence: primer

<400> 92
tatcaagtca gccagggatg 20

<210> 93

<211> 21
<212> DNA
<213> Artificial Sequence

<220>
<223> Description of Artificial Sequence: primer

<400> 93
tgtcaaagta cacatggaaga 21

<210> 94
<211> 20
<212> DNA
<213> Artificial Sequence

<220>
<223> Description of Artificial Sequence: primer

<400> 94
catggtgtag aagtgttgcc 20

<210> 95
<211> 20
<212> DNA
<213> Artificial Sequence

<220>
<223> Description of Artificial Sequence: primer

<400> 95
agcattctag ccaggttcac 20

<210> 96
<211> 21
<212> DNA
<213> Artificial Sequence

<220>
<223> Description of Artificial Sequence: primer

<400> 96
gtgactaact gtgccaaatt c 21

<210> 97
<211> 20
<212> DNA
<213> Artificial Sequence

<220>
<223> Description of Artificial Sequence: primer

<400> 97
cagaactctc gatacttgcg 20

<210> 98
<211> 21
<212> DNA
<213> Artificial Sequence

<220>
<223> Description of Artificial Sequence: primer

<400> 98
gagccaattt acaagatgct g 21

<210> 99
<211> 16
<212> DNA
<213> Artificial Sequence

<220>
<223> Description of Artificial Sequence: primer

<400> 99
tgcccaagat gactgc 16

<210> 100
<211> 20
<212> DNA
<213> Artificial Sequence

<220>
<223> Description of Artificial Sequence: primer

<400> 100
ctggaacatg gctattggac 20

<210> 101
<211> 20
<212> DNA
<213> Artificial Sequence

<220>
<223> Description of Artificial Sequence: primer

<400> 101
tgactcaggg aactcaactc 20

<210> 102
<211> 20
<212> DNA
<213> Artificial Sequence

<220>
<223> Description of Artificial Sequence: primer

<400> 102
ctgggaagag caatgtagac 20

<210> 103
<211> 21
<212> DNA
<213> Artificial Sequence

<220>
<223> Description of Artificial Sequence: primer

<400> 103
tgctttact ctttctctgc c 21

<210> 104
<211> 17
<212> DNA
<213> Artificial Sequence

<220>
<223> Description of Artificial Sequence: primer

<400> 104

cgatgtacag ctgccac 17

<210> 105
<211> 20
<212> DNA
<213> Artificial Sequence

<220>
<223> Description of Artificial Sequence: primer

<400> 105
aagctctgcc aatgactagc 20

<210> 106
<211> 18
<212> DNA
<213> Artificial Sequence

<220>
<223> Description of Artificial Sequence: primer

<400> 106
caaggtgagc tgagaacc 18

<210> 107
<211> 19
<212> DNA
<213> Artificial Sequence

<220>
<223> Description of Artificial Sequence: primer

<400> 107
gccaatcctt tcagaagtt 19

<210> 108
<211> 24
<212> DNA
<213> Artificial Sequence

<220>
<223> Description of Artificial Sequence: primer

<400> 108
ggtagttatt aaaaaggtta ggtg 24

<210> 109
<211> 18
<212> DNA
<213> Artificial Sequence

<220>
<223> Description of Artificial Sequence: primer

<400> 109
tcctgctcta aggtggac 18

<210> 110
<211> 21
<212> DNA
<213> Artificial Sequence

<220>
<223> Description of Artificial Sequence: primer

<400> 110
ttcacatcag caatcaccat g 21

<210> 111
<211> 18
<212> DNA
<213> Artificial Sequence

<220>
<223> Description of Artificial Sequence: primer

<400> 111
accaccacca cctagcgg 18

<210> 112
<211> 21
<212> DNA
<213> Artificial Sequence

<220>
<223> Description of Artificial Sequence: primer

<400> 112
gagaaatatc acagacttggc 21

<210> 113
<211> 18
<212> DNA
<213> Artificial Sequence

<220>
<223> Description of Artificial Sequence: primer

<400> 113
ggagagagtt tgggcaag 18

<210> 114
<211> 22
<212> DNA
<213> Artificial Sequence

<220>
<223> Description of Artificial Sequence: primer

<400> 114
atcatttatt gaactgcaag ag 22

<210> 115
<211> 19
<212> DNA
<213> Artificial Sequence

<220>
<223> Description of Artificial Sequence: primer

<400> 115
catctccagt tctgcaatc 19

<210> 116
<211> 20
<212> DNA
<213> Artificial Sequence

<220>

<223> Description of Artificial Sequence: primer

<400> 116
ttctttcgtg actgcttccg 20

<210> 117
<211> 20
<212> DNA
<213> Artificial Sequence

<220>
<223> Description of Artificial Sequence: primer

<400> 117
aattcacttt gaaccattga 20

<210> 118
<211> 23
<212> DNA
<213> Artificial Sequence

<220>
<223> Description of Artificial Sequence: primer

<400> 118
gctactctga gtctgaagat tgt 23

<210> 119
<211> 20
<212> DNA
<213> Artificial Sequence

<220>
<223> Description of Artificial Sequence: primer

<400> 119
agtttgttct ctccttcacg 20

<210> 120
<211> 20
<212> DNA
<213> Artificial Sequence

<220>
<223> Description of Artificial Sequence: primer

<400> 120
tcttctgtgg aataattccc 20

<210> 121
<211> 17
<212> DNA
<213> Artificial Sequence

<220>
<223> Description of Artificial Sequence: primer

<400> 121
tgcacttgag ggaagga 17

<210> 122
<211> 22
<212> DNA
<213> Artificial Sequence

<220>
<223> Description of Artificial Sequence: primer

<400> 122
ggattttagc ttttctattg gc 22

<210> 123
<211> 21
<212> DNA
<213> Artificial Sequence

<220>
<223> Description of Artificial Sequence: primer

<400> 123
tttagtgcag accaatattc c 21

<210> 124
<211> 22
<212> DNA
<213> Artificial Sequence

<220>
<223> Description of Artificial Sequence: primer

<400> 124
ctttcataga gttcatagcc ac 22

<210> 125
<211> 17
<212> DNA
<213> Artificial Sequence

<220>
<223> Description of Artificial Sequence: primer

<400> 125
accgctgtac actgcag 17

<210> 126
<211> 22
<212> DNA
<213> Artificial Sequence

<220>
<223> Description of Artificial Sequence: primer

<400> 126
catatagtca ggaaatctag gg 22

<210> 127
<211> 21
<212> DNA
<213> Artificial Sequence

<220>
<223> Description of Artificial Sequence: primer

<400> 127
tctaaattga tgccaagtac a 21

<210> 128
<211> 23
<212> DNA

<213> Artificial Sequence

<220>
<223> Description of Artificial Sequence: adaptor

<220>
<221> misc_feature
<222> (23)
<223> complementary strand has a single stranded DNA "3'-ctag-5'" which extends from the position in the direction of 3'→5'.

<400> 128
gtacatattg tcgttagaac gcg 23

<210> 129
<211> 26
<212> DNA
<213> Artificial Sequence

<220>
<223> Description of Artificial Sequence: adaptor

<220>
<221> misc_feature
<222> (26)
<223> complementary strand has a single stranded DNA "3'-ctag-5'" which extends from the position in the direction of 3'→5'.

<400> 129
gtacatattg tcgttagaac gcgact 26

<210> 130
<211> 29
<212> DNA
<213> Artificial Sequence

<220>
<223> Description of Artificial Sequence: adaptor

<220>
<221> misc_feature
<222> (29)
<223> complementary strand has a single stranded DNA "3'-ctag-5'"

which extends from the position in the direction of 3'→5'.

<400> 130
gtacatattg tcgttagaac gcgcatact 29

<210> 131
<211> 32
<212> DNA
<213> Artificial Sequence

<220>
<223> Description of Artificial Sequence: adaptor

<220>
<221> misc_feature
<222> (32)
<223> complementary strand has a single stranded DNA "3'-ctag-5'" which extends from the position in the direction of 3'→5'.

<400> 131
gtacatattg tcgttagaac gcgatccata ct 32

<210> 132
<211> 35
<212> DNA
<213> Artificial Sequence

<220>
<223> Description of Artificial Sequence: adaptor

<220>
<221> misc_feature
<222> (35)
<223> complementary strand has a single stranded DNA "3'-ctag-5'" which extends from the position in the direction of 3'→5'.

<400> 132
gtacatattg tcgttagaac gcgtcaatcc atact 35

<210> 133
<211> 38
<212> DNA
<213> Artificial Sequence

<220>
<223> Description of Artificial Sequence: adaptor

<220>
<221> misc_feature
<222> (38)
<223> complementary strand has a single stranded DNA "3'-ctag-5'" which extends from the position in the direction of 3'→5'.

<400> 133
gtacatattg tcgttagaac gcgtactcaa tccatact 38

<210> 134
<211> 22
<212> DNA
<213> Artificial Sequence

<220>
<223> Description of Artificial Sequence: primer

<400> 134
gtacatattg tcgttagaac gc 22

<210> 135
<211> 20
<212> DNA
<213> Artificial Sequence

<220>
<223> Description of Artificial Sequence: primer

<400> 135
ccttctcagt gggcatgatt 20

<210> 136
<211> 20
<212> DNA
<213> Artificial Sequence

<220>
<223> Description of Artificial Sequence: primer

<400> 136
accggccaat cacttacaac 20

<210> 137
<211> 20
<212> DNA
<213> Artificial Sequence

<220>
<223> Description of Artificial Sequence: primer

<400> 137
tccaatctgg gaggaaactg 20

<210> 138
<211> 20
<212> DNA
<213> Artificial Sequence

<220>
<223> Description of Artificial Sequence: primer

<400> 138
ggtgtccgac agttctggtt 20

<210> 139
<211> 20
<212> DNA
<213> Artificial Sequence

<220>
<223> Description of Artificial Sequence: primer

<400> 139
tcttggtatg gggacaggag 20

<210> 140
<211> 20
<212> DNA
<213> Artificial Sequence

<220>
<223> Description of Artificial Sequence: primer

<400> 140
gttcagacag ggctgcttgt 20

<210> 141
<211> 20
<212> DNA
<213> Artificial Sequence

<220>
<223> Description of Artificial Sequence: primer

<400> 141
acctgcattg tcattgctca 20

<210> 142
<211> 20
<212> DNA
<213> Artificial Sequence

<220>
<223> Description of Artificial Sequence: primer

<400> 142
ctcatgggtc ccttttttcaa 20

<210> 143
<211> 20
<212> DNA
<213> Artificial Sequence

<220>
<223> Description of Artificial Sequence: primer

<400> 143
tcgcctagaa tctgcattcc 20

<210> 144
<211> 20
<212> DNA
<213> Artificial Sequence

<220>

<223> Description of Artificial Sequence: primer

<400> 144
aactcaaatg tccccactcg 20

<210> 145
<211> 20
<212> DNA
<213> Artificial Sequence

<220>
<223> Description of Artificial Sequence: primer

<400> 145
gaatgcacga agacatcgaa 20

<210> 146
<211> 20
<212> DNA
<213> Artificial Sequence

<220>
<223> Description of Artificial Sequence: primer

<400> 146
gtgtgcggca tagacacact 20

<210> 147
<211> 20
<212> DNA
<213> Artificial Sequence

<220>
<223> Description of Artificial Sequence: primer

<400> 147
ggccattcta tgccttcaaa 20

<210> 148
<211> 20
<212> DNA
<213> Artificial Sequence

<220>
<223> Description of Artificial Sequence: primer

<400> 148
tcaggtgggg aaactgagac 20

<210> 149
<211> 20
<212> DNA
<213> Artificial Sequence

<220>
<223> Description of Artificial Sequence: primer

<400> 149
agggcactat ccgaaaggtt 20

<210> 150
<211> 20
<212> DNA
<213> Artificial Sequence

<220>
<223> Description of Artificial Sequence: primer

<400> 150
aggctgccag tctggagtaa 20

<210> 151
<211> 20
<212> DNA
<213> Artificial Sequence

<220>
<223> Description of Artificial Sequence: primer

<400> 151
gtatacctcc cctgcccagt 20

<210> 152
<211> 20
<212> DNA
<213> Artificial Sequence

<220>
<223> Description of Artificial Sequence: primer

<400> 152
cctgggtttg agtcgtctgt 20

<210> 153
<211> 20
<212> DNA
<213> Artificial Sequence

<220>
<223> Description of Artificial Sequence: primer

<400> 153
gagaaaacag gagcgacagc 20

<210> 154
<211> 20
<212> DNA
<213> Artificial Sequence

<220>
<223> Description of Artificial Sequence: primer

<400> 154
gcacacaacc aagggaatct 20

<210> 155
<211> 20
<212> DNA
<213> Artificial Sequence

<220>
<223> Description of Artificial Sequence: primer

<400> 155
gagggtggat ggtgttgagt 20

<210> 156
<211> 20
<212> DNA

<213> Artificial Sequence

<220>
<223> Description of Artificial Sequence: primer

<400> 156
gagcctctgc cctacctctt 20

<210> 157
<211> 20
<212> DNA
<213> Artificial Sequence

<220>
<223> Description of Artificial Sequence: primer

<400> 157
gggtatgtgt gggtttgagg 20

<210> 158
<211> 20
<212> DNA
<213> Artificial Sequence

<220>
<223> Description of Artificial Sequence: primer

<400> 158
gccgtacagc atgtacttcg 20

<210> 159
<211> 20
<212> DNA
<213> Artificial Sequence

<220>
<223> Description of Artificial Sequence: primer

<400> 159
acaagggcta aaggggacat 20

<210> 160
<211> 20

<212> DNA
<213> Artificial Sequence

<220>
<223> Description of Artificial Sequence: primer

<400> 160
cagttgcgta gggaggtcat 20

<210> 161
<211> 20
<212> DNA
<213> Artificial Sequence

<220>
<223> Description of Artificial Sequence: primer

<400> 161
gagctcttct gtgcccagtc 20

<210> 162
<211> 20
<212> DNA
<213> Artificial Sequence

<220>
<223> Description of Artificial Sequence: primer

<400> 162
aggacgctgt catgctcttt 20

<210> 163
<211> 20
<212> DNA
<213> Artificial Sequence

<220>
<223> Description of Artificial Sequence: primer

<400> 163
aggtaccaac ctggcttgtg 20

<210> 164

<211> 20
<212> DNA
<213> Artificial Sequence

<220>
<223> Description of Artificial Sequence: primer

<400> 164
tctgaaaatc tcggccttgt 20

<210> 165
<211> 20
<212> DNA
<213> Artificial Sequence

<220>
<223> Description of Artificial Sequence: primer

<400> 165
gccaagactg cacacaagaa 20

<210> 166
<211> 20
<212> DNA
<213> Artificial Sequence

<220>
<223> Description of Artificial Sequence: primer

<400> 166
cttgggtttc acccactcat 20

<210> 167
<211> 20
<212> DNA
<213> Artificial Sequence

<220>
<223> Description of Artificial Sequence: primer

<400> 167
tcagcttcca acatgctacg 20

<210> 168
<211> 20
<212> DNA
<213> Artificial Sequence

<220>
<223> Description of Artificial Sequence: primer

<400> 168
cttgccaacc ttgatcacct 20

<210> 169
<211> 20
<212> DNA
<213> Artificial Sequence

<220>
<223> Description of Artificial Sequence: primer

<400> 169
tgggtgatgg actcttcctc 20

<210> 170
<211> 20
<212> DNA
<213> Artificial Sequence

<220>
<223> Description of Artificial Sequence: primer

<400> 170
gctgcattgt tgtgttgtcc 20

<210> 171
<211> 20
<212> DNA
<213> Artificial Sequence

<220>
<223> Description of Artificial Sequence: primer

<400> 171
acccagccaa tacaaactgc 20

<210> 172
<211> 20
<212> DNA
<213> Artificial Sequence

<220>
<223> Description of Artificial Sequence: primer

<400> 172
tgagatttg ctcggttgtg 20

<210> 173
<211> 20
<212> DNA
<213> Artificial Sequence

<220>
<223> Description of Artificial Sequence: primer

<400> 173
tggaccctga ctcttgtgtg 20

<210> 174
<211> 20
<212> DNA
<213> Artificial Sequence

<220>
<223> Description of Artificial Sequence: primer

<400> 174
aagggatgga ggagcaaagt 20

<210> 175
<211> 20
<212> DNA
<213> Artificial Sequence

<220>
<223> Description of Artificial Sequence: primer

<400> 175

gttcagtagt cgcggttggt 20

<210> 176
<211> 20
<212> DNA
<213> Artificial Sequence

<220>
<223> Description of Artificial Sequence: primer

<400> 176
gaagtggtgc tgatggtcct 20

<210> 177
<211> 20
<212> DNA
<213> Artificial Sequence

<220>
<223> Description of Artificial Sequence: primer

<400> 177
ttcctgcttc gtgtgttgtc 20

<210> 178
<211> 20
<212> DNA
<213> Artificial Sequence

<220>
<223> Description of Artificial Sequence: primer

<400> 178
tcaaaggatg agggaagacg 20

<210> 179
<211> 20
<212> DNA
<213> Artificial Sequence

<220>
<223> Description of Artificial Sequence: primer

<400> 179
tgtgcgatcg ttactgcttt 20

<210> 180
<211> 20
<212> DNA
<213> Artificial Sequence

<220>
<223> Description of Artificial Sequence: primer

<400> 180
agggcttcag aggcttcttc 20

<210> 181
<211> 20
<212> DNA
<213> Artificial Sequence

<220>
<223> Description of Artificial Sequence: primer

<400> 181
ccatcgtggg tgctattctt 20

<210> 182
<211> 20
<212> DNA
<213> Artificial Sequence

<220>
<223> Description of Artificial Sequence: primer

<400> 182
aggagatgtg ctccgacact 20

<210> 183
<211> 20
<212> DNA
<213> Artificial Sequence

<220>
<223> Description of Artificial Sequence: primer

<400> 183
aacgctctga agtcctggaa 20

<210> 184
<211> 20
<212> DNA
<213> Artificial Sequence

<220>
<223> Description of Artificial Sequence: primer

<400> 184
gtccacattg gccctgtagt 20

<210> 185
<211> 20
<212> DNA
<213> Artificial Sequence

<220>
<223> Description of Artificial Sequence: primer

<400> 185
aaaaatgcag accacgaagg 20

<210> 186
<211> 20
<212> DNA
<213> Artificial Sequence

<220>
<223> Description of Artificial Sequence: primer

<400> 186
ctactcagga gggaggcaga 20

<210> 187
<211> 20
<212> DNA
<213> Artificial Sequence

<220>

<223> Description of Artificial Sequence: primer

<400> 187
gctgacgtgc tgcagaatta 20

<210> 188
<211> 20
<212> DNA
<213> Artificial Sequence

<220>
<223> Description of Artificial Sequence: primer

<400> 188
atcctggcac tctgctcact 20

<210> 189
<211> 20
<212> DNA
<213> Artificial Sequence

<220>
<223> Description of Artificial Sequence: primer

<400> 189
tttcttcagc ctgtggaagg 20

<210> 190
<211> 20
<212> DNA
<213> Artificial Sequence

<220>
<223> Description of Artificial Sequence: primer

<400> 190
gacgcttgta ggccttgttc 20

<210> 191
<211> 20
<212> DNA
<213> Artificial Sequence

<220>
<223> Description of Artificial Sequence: primer

<400> 191
accggccaat cacttacaac 20

<210> 192
<211> 20
<212> DNA
<213> Artificial Sequence

<220>
<223> Description of Artificial Sequence: primer

<400> 192
cgtacaatgg gatgcagatg 20

## Claims

1. A gene panel comprising names of genes of which expression levels change in hepatocytes during liver regeneration as compared with those in a normal state and expression profiles of the genes.

2. The gene panel according to claim 1, wherein the changes of the gene expression levels are changes in the expression levels in a model animal after partial hepatectomy as compared with the expression levels in a normal state in the model animal.

3. The gene panel according to claim 1 or 2, wherein the expression profiles include expression profiles over time during liver regeneration.

4. The gene panel according to any one of claims 1 to 3, which includes sequence information of a group of PCR primers for analyzing the expression profiles of the genes.

5. The gene panel according to any one of claims 2 to 4, wherein the model animal is a rat.

6. The gene panel according to any one of claims 1 to 5, which includes expression profiles of at least 6 types of genes among 166 types of the genes represented by the numbers 1 to 166 in Tables 1, 6 and 7.

7. The gene panel according to claim 6, wherein the at least 6 types of genes are selected from 151 types of the genes represented by numbers 1 to 151 in Tables 1 and 6.

8. The gene panel according to claim 6, wherein the at least 6 types of genes are selected from 137 types of the genes represented by the numbers 1 to 137 in Tables 1 and 6.

9. The gene panel according to any one of claims 6 to 8, wherein the at least 6 types of genes are the genes represented by the numbers 5, 17, 36, 46, 67 and 68 in Table 1.

10. A method for producing a gene panel comprising expression profiles of genes of which expression levels change in hepatocytes during liver regeneration as compared with those in a normal state, which comprises the steps of:

    (a) measuring expression levels of various genes in hepatocytes of a model animal in a normal state and expression levels of the genes during liver regeneration;
    (b) comparing the expression levels, respectively; and
    (c) identifying a group of genes of which expression levels change during liver regeneration and producing expression profiles from information about gene names and changes in the expression levels.

11. The method according to claim 10, wherein, in the step (a), the expression levels of the gene are analyzed over time during liver regeneration.

12. The method according to claim 11, wherein a liver regeneration accelerating substance is administered before, during or after liver regeneration.

13. The method according to claim 12, wherein the liver regeneration accelerating substance is L-alanine.

14. The method according to any one of claims 10 to 13, wherein the gene expression levels are analyzed by one or more kinds of methods selected from the gene chip method, the ATAC-PCR method and the Taqman PCR (SYBR Green) method.

15. The method according to claim 14, wherein the gene expression levels are analyzed by the gene chip method and the ATAC-PCR method.

16. The method according to claim 14, wherein the gene expression levels are analyzed by the Taqman PCR (SYBR Green) method.

17. A method for screening for a drug involved in liver regeneration, which comprises administering a drug to a model animal or liver tissue or cells and profiling expressions of genes constituting the gene panel according to claim 1.

**18.** A method for evaluating a condition of liver, which comprises profiling expressions of genes constituting the gene panel according to claim 1 for liver of a subject.

**19.** A group of primers used in the method for producing a gene panel according to claim 10, the screening method according to claim 17 or the evaluation method according to claim 18, which comprises all or a part of the oligonucleotides of SEQ ID NOS: 1 to 127 and 135 to 192.

*Fig. 1*

| | | |
|---|---|---|
| *MboI* | ***CT | 3' |
| | ***GACTAG | 5' |
| *NlaIII* | ***CTCATG | 3' |
| | ***GA | 5' |
| *BfaI* | ***CTC | 3' |
| | ***GAGAT | 5' |
| *HpaII* | ***CTC | 3' |
| | ***GAGGC | 5' |

*Fig.2*

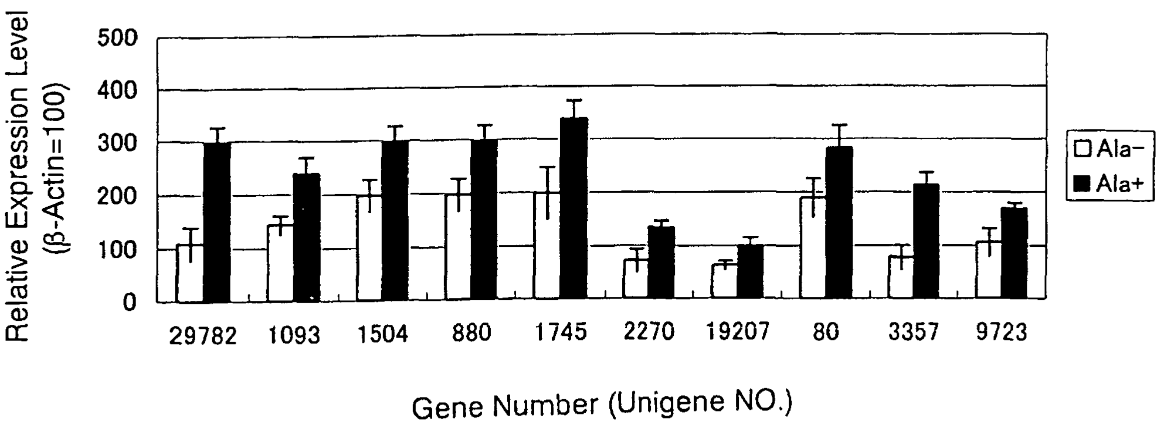

*Fig.3*

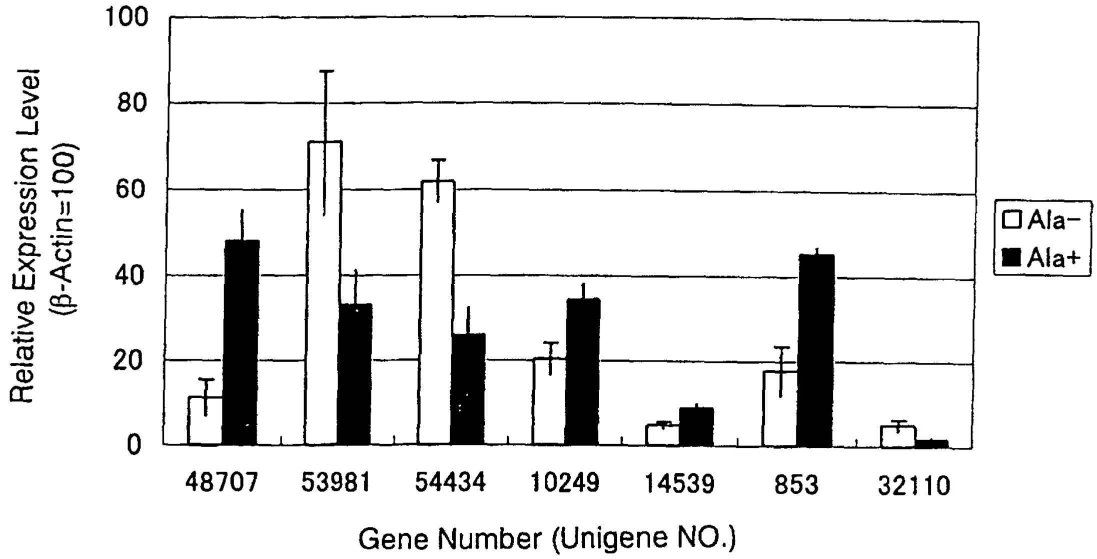
100
80
60
40
20
0
Relative Expression Level
(β-Actin=100)
Ala-
Ala+
48707
53981
54434
10249
14539
853
32110
Gene Number (Unigene NO.)

*Fig.4*

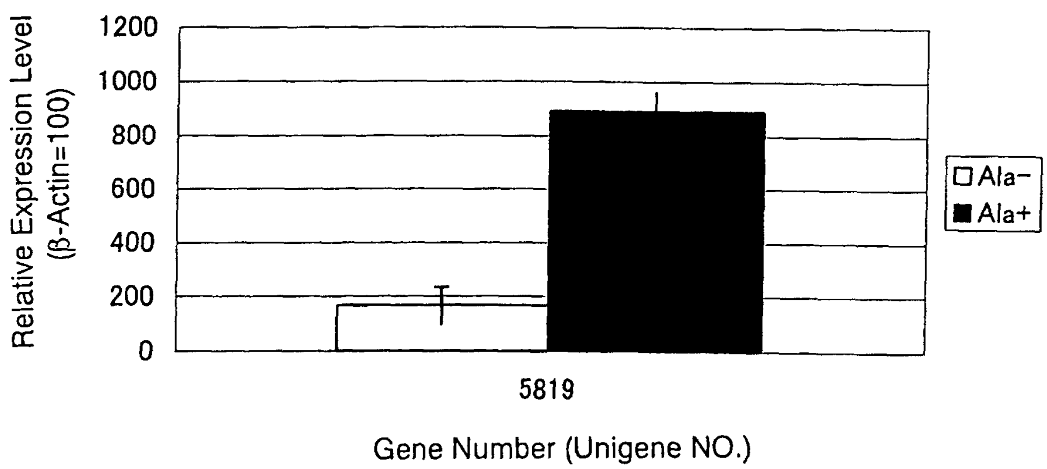
1200
1000
800
600
400
200
0
Relative Expression Level
(β-Actin=100)
Ala-
Ala+
5819
Gene Number (Unigene NO.)

## INTERNATIONAL SEARCH REPORT

International application No.
PCT/JP02/02372

A. CLASSIFICATION OF SUBJECT MATTER
Int.Cl$^7$ C12N15/09, C12Q1/68, G01N33/15, 33/50, 37/00

According to International Patent Classification (IPC) or to both national classification and IPC

B. FIELDS SEARCHED

Minimum documentation searched (classification system followed by classification symbols)
Int.Cl$^7$ C12N15/09, C12Q1/68, G01N33/15, 33/50, 37/00

Documentation searched other than minimum documentation to the extent that such documents are included in the fields searched

Electronic data base consulted during the international search (name of data base and, where practicable, search terms used)
JICST FILE(JOIS), MEDLINE(STN), WPI(DIALOG), BIOSIS(DIALOG)

C. DOCUMENTS CONSIDERED TO BE RELEVANT

| Category* | Citation of document, with indication, where appropriate, of the relevant passages | Relevant to claim No. |
|---|---|---|
| Y | XU, W., et al., Identification and characterization Of differentially expressed genes in the early response phase during liver regeneration., Biochem Biophys Res Commun., 19 November, 2000 (19.11.00), Vol.278, No.2, pages 318 to 325. | 1-19 |
| Y | KAR, S., et al., Differential display and cloning of messenger RNAs from the late phase of rat liver regeneration., Biochem Biophys Res Commun., 06 July, 1995(06.07.95), Vol.212, No.1, pages 21 to 26 | 1-19 |
| Y | RININGER, J. A., et al., Time course comparison of cell-cycle protein expression following partial hepatectomy and WY14, 643-induced hepatic cell proliferation in F344 rats., Carcinogenesis, 1997 May, Vol.18, No.5, pages 935 to 941 | 1-19 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 June, 2002 (06.06.02) | 25 June, 2002 (25.06.02) |
| Name and mailing address of the ISA/ Japanese Patent Office | Authorized officer |
| Facsimile No. | Telephone No. |

Form PCT/ISA/210 (second sheet) (July 1998)

INTERNATIONAL SEARCH REPORT

International application No.
PCT/JP02/02372

C (Continuation). DOCUMENTS CONSIDERED TO BE RELEVANT

| Category* | Citation of document, with indication, where appropriate, of the relevant passages | Relevant to claim No. |
|---|---|---|
| Y | BENNETT, L. M., et al., Strain-dependent differences in DNA synthesis and gene expression in the regenerating livers of CB57BL/6J and C3H/HeJ mice., Mol Carcinog., 1995 September, Vol.14, No.1, pages 46 to 52 | 1-19 |
| Y | KATO, K., Adaptor-tagged competitive PCR: a novel method for measuring relative gene expression., Nucleic Acids Res., 15 November, 1997 (15.11.97), Vol.25, No.22, pages 4694 to 4696 | 1-19 |
| Y | SCHMITTGEN, T. D., et al., Quantitative reverse transcription-polymerase chain reaction to study mRNA decay: comparison of endpoint and real-time methods., Anal Biochem., 15 October, 2000 (15.10.00), Vol.285, No.2, pages 194 to 204 | 1-19 |
| Y | JP 5-229940 A (Ajinomoto Co., Inc.), 07 September, 1993 (07.09.93), (Family: none) | 1-19 |

Form PCT/ISA/210 (continuation of second sheet) (July 1998)